(19) Europäisches Patentamt

European Patent Office

Office européen des brevets

(11) **EP 3 260 110 B1**

(12) **EUROPEAN PATENT SPECIFICATION**

(45) Date of publication and mention of the grant of the patent:
**22.04.2020 Bulletin 2020/17**

(51) Int Cl.:
*A61K 8/04* *(2006.01)*       *A61K 8/19* *(2006.01)*
*A61K 8/33* *(2006.01)*       *A61K 8/81* *(2006.01)*
*A61K 8/87* *(2006.01)*       *A61Q 5/06* *(2006.01)*
*B05B 1/34* *(2006.01)*       *B65D 83/14* *(2006.01)*

(21) Application number: **17186155.2**

(22) Date of filing: **06.12.2011**

(54) **AEROSOL HAIRSPRAY PRODUCT FOR STYLING AND/OR SHAPING HAIR**

AEROSOLHAARSPRAYPRODUKT ZUM STYLEN UND/ODER FORMEN VON HAAR

PRODUIT DE LAQUE AÉROSOL POUR COIFFER ET/OU METTRE EN FORME LES CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2011 EP 11007522**

(43) Date of publication of application:
**27.12.2017 Bulletin 2017/52**

(62) Document number(s) of the earlier application(s) in accordance with Art. 76 EPC:
**11192063.3 / 2 570 113**

(73) Proprietor: **The Procter & Gamble Company Cincinnati, OH 45202 (US)**

(72) Inventors:
• **BIRKEL, Susanne**
  **64295 Darmstadt (DE)**
• **CHRISTOPOULOU, Wassiliki**
  **64347 Darmstadt (DE)**
• **DAL BÒ, Paolo**
  **2611 DN Delft (NL)**
• **GÄNGER, Klaus**
  **64319 Pfungstadt (DE)**
• **GIESEN, Bettina**
  **65474 Bischofsheim (DE)**
• **SMITH, Scott**
  **Cincinnati, Ohio 45247 (US)**
• **BURGHAUS, Johannes**
  **64823 Gross-Umstadt (DE)**

(74) Representative: **P&G Patent Germany Procter & Gamble Service GmbH Sulzbacher Straße 40 65824 Schwalbach am Taunus (DE)**

(56) References cited:
**EP-A1- 0 758 545       WO-A1-89/05195
WO-A1-03/061839       DE-U1- 29 707 765
US-A- 5 304 368**

• **Data sheet on Luvimer® 100P, Luvimer® 36D, Luvimer® 30E**
• **Datasheet on Amphomer®**
• **Datasheet on Balance® CR**

Remarks:
The file contains technical information submitted after the application was filed and not included in this specification

**Description**

FIELD OF THE INVENTION

An aerosol hairspray product for styling and/or shaping hair.

BACKGROUND OF THE INVENTION

Hairstyling products such as hairsprays are used for achieving different hairstyles and for holding hair strands in place for a period of time. Typically, hairsprays comprise film-forming polymers, which when applied to keratin-containing fibres, such as human hair, form fibre-fibre welds. These welds 'glue' the fibres together and hence impart hold to the hairstyle.

Aerosol hairspray products usually comprise a pressure-resistant container, a nozzle, a propellant, and a hairstyling formulation. A hairspray composition is normally ejected from such products via aerosol-forming nozzle. See, for example, US2009/0104138A1. Commonly used propellants include the volatile organic compounds (VOCs) propane, butane, 1,1-difluoroethane, and dimethylether. However, VOCs are known to react with certain nitrogenic oxides, which in turn may result in the formation of ground-level ozone - a potential source of health problems. Alcohols are also often used in the hairstyling formulation, for example to reduce surface tension. However, a high proportion of alcohol may leave the hair feeling dry and brittle and some alcohols may cause an allergic response in some users. Also, ethanol is flammable and is a VOC.

European patent application EP 0 758 545 A1 is related to an aqueous or hydroalcoholic cosmetic composition, particularly in the form of an aerosol, comprising an aqueous dispersion of insoluble particles of a filmogenic polymer and an insoluble silicone.

Utility model DE 297 07 765 U1 refers to a hair treatment agent, in particular a hair styling composition in the form of a pump spray, with conditioning properties, which gives the hair in particular improved combability and causes a pleasant feel and a natural appearance.

US Patent US 5,304,368 A1 sets out a non-foaming, non-viscous, alcohol-free, water-based pressurized hair-fixative spray product for use as the total fill in an aerosol container by delivery from an actuated-valve of predetermined dimensions, particularly the vapor tap and stem orifice sizes.

There is a constant need, therefore, for more environmentally friendly, more sustainable, and affordable hairspray products, in particular for aerosol hairspray products comprising low levels of VOC and alcohol. However, altering one or more features of an aerosol hairspray product can be challenging since the interrelationship therebetween affects the product performance. For example, utilising a different propellant may result in an unacceptable droplet size of the ejected composition and consequently unsatisfactory hold. Furthermore, certain hairstyling polymers may be incompatible with hairspray products comprising low levels of VOC and/or alcohol.

When considering the aforementioned needs, therefore, good hairspray performance should be maintained. Performance benefits may include, for example: excellent hold; long-lasting hold; good humidity resistance; shapeable hold; acceptable drying time; excellent soft, natural hair feel; acceptable and/or non-stickiness/tackiness of the hands and hair. Of particular relevance to consumers is natural hair feel and non-tackiness of the hands and hair.

SUMMARY OF THE INVENTION

In a first aspect, the invention relates to an aerosol hairspray product (120) for styling and/or shaping hair wherein the product (120) comprises:

    i. a container comprising a container wall which encloses a reservoir (122) for storing a hairstyling formulation and a propellant;
    ii. the hairstyling formulation comprising:

        (a) at least 50% water by total weight of the hairstyling formulation and propellant; and
        (b) from 0.01% to 20% of a hairstyling polymer by total weight of the hairstyling formulation and propellant, wherein the hairstyling polymer is selected from the group consisting of: acrylates copolymers of two or more monomers of (meth)acrylic acid or one of their simple esters; octylacrylamide/acrylate/butylaminoethyl methacrylate copolymers; acrylates/hydroxyesters acrylates copolymers of butyl acrylate, methyl methacrylate, methacrylic acid, ethyl acrylate and hydroxyethyl methacrylate; polyurethane-14/AMP-acrylates polymer blend; and mixtures thereof, wherein the hairstyling polymer is at least 60%, or at least 80% neutralized; and

    iii. a propellant, which is selected from the group consisting of compressed gas propellants, liquefied gas propellants, and mixtures thereof; and
iv. a spraying device attached to the container for dispensing the hairstyling formulation from the reservoir (122) of the container;

and wherein the product (120) comprises 2% or less alcohol by total weight of the hairstyling formulation and propellant, or is less than 1% of alcohol;
wherein the product (120) comprises less than 15% volatile organic compound by total weight of the hairstyling formulation and propellant.

In a second aspect, the invention relates to a method for styling hair comprising:

i. applying to hair an ejected composition, wherein the ejected composition is ejected by the hairspray product (120) according to the first aspect;
ii. drying the ejected composition on the hair.

In a third aspect, the invention relates to the use of the product (120) according to the first aspect for fixing and/or shaping a hairstyle.

DETAILED DESCRIPTION OF THE INVENTION

All percentages are by weight of the total composition/formulation, unless stated otherwise. All ratios are weight ratios, unless stated otherwise. All ranges are inclusive and combinable. The number of significant digits conveys neither a limitation on the indicated amounts nor on the accuracy of the measurements. The term "molecular weight" or "M.Wt." as used herein refers to the weight average molecular weight unless otherwise stated. "QS" or "QSP" means sufficient quantity for 100%. +/- indicates the standard deviation.

Embodiments and aspects described herein may comprise or be combinable with elements or components of other embodiments and/or aspects despite not being expressly exemplified in combination, unless otherwise stated or an incompatibility is stated.

The term "aerosol" as used herein, means a suspension of fine droplets in a gas. The aerosol hairspray product atomises the hairspray formulation i.e. creates an aerosol. Due to surface tension, droplets are normally substantially spherical. As used herein, the "droplet size" is defined as the median diameter of ejected droplets.

The term "aerosol hairspray product" does not encompass mousse or foam products. The term "mousse" or "foam" as defined herein means a dispersion of gas bubbles in a liquid. Commonly, mousse or foam compositions usually comprise greater than 0.3% surfactant by weight. The surfactant results in the formation of spherical bubbles which form the mousse or foam consistency. However, foams and mousses can also be formed from surfactant-free formulations via other means, for example special actuators, using proteins e.g. egg white protein. Typically, hairstyling products that eject a mousse/foam also comprise from 6% to 16% by weight propellant.

The term "aerosol hairspray product" does not encompass gel products or products comprising or ejecting a gel composition. Gels may be dispensed via a pump spray actuator. Hand gel formulations typically have a viscosity of from 8,000 mPa·s to 20,000 mPa·s depending on the desired performance. The ejected composition of spray gels typically has a droplet size of at least 80 micron in diameter.

As used herein, the term "on-hair drying time" means the amount of time it takes for the ejected composition to dry on the hair. The on-hair drying time is measured by spraying a specific pattern on the hair and then timing when the hair ceases to feel tacky and damp in the hand.

As used herein, the term "ejection flow" is defined as the loss in total weight of the aerosol hairspray product after 5 seconds of spraying. This value is normally divided by 5 to give grams per sec. The ejection flow should achieve a balance between excellent hold and sufficiently fast drying time. For example, if too much ejected composition is applied to the hair in a short period, then the on-hair drying time may be unacceptably long.

Herein, "comprising" means that other steps and other ingredients which do not affect the end result can be added. This term encompasses the terms "consisting of' and "consisting essentially of". The compositions, methods, uses, and processes herein can comprise, consist of, and consist essentially of the elements and limitations of the invention described herein, as well as any of the additional or optional ingredients, components, steps, or limitations described herein.

The term "polymer" as used herein shall include all materials made by the polymerisation of monomers as well as natural polymers. Polymers made from only one type of monomer are called homopolymers. A polymer comprises at least two monomers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

The term "hairstyling polymer" as used herein means hair-fixing polymers which form films on a surface. In the context of hair, this surface is the surface of individual hair fibres or a plurality thereof. The polymer causes them to be glued together to build welds, which are crosslinks that provide the hold benefit. In concert, these welds form a 'hairnet' to provide hair hold and volume benefits to the user. When the net of welds is effectively formed, the hold and volume benefits can last all day and offer good resistance to environmental humidity.

The hairspray product according to the present invention is suitable for application onto human hair. The term "suitable for application to human hair" as used herein means that the compositions or components thereof so described are suitable for use in contact with human hair and the scalp without undue toxicity, incompatibility, instability, allergic response, and the like.

The term "maximum incremental reactivity" value or "MIR" value as defined herein, means a measure of the increase in ozone formation per unit weight of a hydrocarbon when added to the atmosphere. Hence, MIR measured the ozone forming potential of a compound. A similar measurement to MIR is "photochemical ozone creation potential" or "POCP".

The term "global warming potential" or "GWP" as defined herein is a measure of how much a given mass of a compound is calculated to contribute to global warming compared to that of the same mass of carbon dioxide. The global warming potential of carbon dioxide, therefore, is 1. As used herein, the GWP values are those calculated for a 100 year time horizon, unless otherwise stated.

As used herein, the term "volatile organic compound" or "VOC", as used herein means any organic compound having an initial boiling point less than or equal to 250°C measured at a standard pressure of 101.3 kPa. In an embodiment, "VOC" means any compound having a vapour pressure of 0.01 kPa or more at 293.15 K (i.e. 20°C). "Organic" as used herein means any compound containing at least the element carbon and one or more of hydrogen, halogen, oxygen, sulfur, phosphorus, silicon, or nitrogen. Certain volatile compounds of organic chemistry falling within this definition are known to photochemically react with nitrogenic oxides in the presence of sunlight and, in turn, this produces ground-level ozone and photochemical smog. In fact, in the United States, the definition of VOC for US legislative purposes (U.S. EPA 40 CFR 51. 100[s]) defines only those organic compounds without negligible photochemical reactivity. Examples of compounds considered to be VOCs for the purposes of this application include: ethanol, dimethylether, 1,1-difluoroethane, 1,1,1,2-tetrafluoroethane, pentane, *n*-butane, *iso*-butane, propane, *trans*-1,3,3,3-tetrafluoropropene, free formic acid (i.e. not its salt). Certain fragrances and plant extracts are also VOCs.

The term "non-flammable", as used herein in terms of the aerosol hairspray product, means the product contains 1% or less flammable components and the chemical heat of combustion is less than 20 kJ/g and is also considered non-flammable following an ignition distance test and, if necessary, the enclosed space test. If the chemical heat of combustion is less than 20 kJ/g, then the aerosol is classified as flammable if ignition occurs at a distance of 15 cm or more. The ignition distance test for spray aerosols is a standard test wherein the aerosol is sprayed in the direction of an ignition source at intervals of 15 cm to observe if ignition and sustained combustion takes place. Ignition and sustained combustion is defined as when a stable flame is maintained for at least 5 seconds. The ignition source is defined as a gas burner with a blue, non-luminous flame 4-5 cm in height. If no ignition occurs in the ignition distance test, the enclosed space test shall be performed and in this case, the aerosol is classified as flammable if the time equivalent is less than or equal to 300 s/m$^3$ or the deflagration density is less than or equal to 300 g/m$^3$; otherwise the aerosol is classified as non-flammable. The enclosed space ignition test is a standard test wherein the contents of an aerosol dispenser are sprayed into a cylindrical test vessel containing a burning candle. If an observable ignition occurs, the elapsed time and amount discharged is noted. These definitions are that of the UN Manual of Tests and Criteria, Part III, Section 31. The chemical heat of combustion can be determined via the standard method ASTM D 240.

The term "substantially free from", "substantially free of', or grammatical equivalents thereof, as defined herein means less than 1%, or less than 0.8%, or less than 0.5%, or less than 0.3%, or 0%.

The inventors have surprisingly overcome the above hindrances and answered the aforementioned needs by carefully selecting the specific combination of mutually compatible features such that the interaction therebetween results in a hairspray with good performance. Firstly, the hairspray formulation pursuant to the present invention is water-based i.e. it is an aqueous system rather than a predominantly alcohol-based system as is conventionally employed. This water-based system provides safety, sustainability, environmental and cost advantages. Secondly, the hairspray product comprises 2% or less alcohol by total weight of the hairstyling formulation and propellant, or is substantially free of alcohol. This additionally provides highly consumer-relevant benefits since alcohol has the reputation of causing the hair to become brittle and dry, particularly for naturally fine and/or dry hair. Without being bound by theory it is believed that low alcohol concentrations help to reduce drying out effect i.e. reduce a perceived brittle, harsh feel of the hair. Consumers prefer a more natural hair feel and look. The inventors have found that selected hairstyling polymers are particularly suited for use in such hairspray formulation. The inventors have found that hairstyling polymer M.Wt., glass transition temperature, water-compatibility and chemistry are important factors in order to create a low viscosity, fully dissolved, readily sprayable hairstyling formulation that provides an ejected composition that, following normal application onto hair, results in good hairstyle hold, good humidity resistance, no residues or flaking on hair, and yet is easily washed out. The selected hairstyling polymers meet these criteria - in particular, the hairstyling polymers, despite being highly compatible in the water-based hairstyling formulation, also provide humidity resistance in high relative humidity for the hairstyle and yet are easily washed out with normal shampoo. Moreover, the hold offered by the selected hairstyling polymers is good, but without causing a 'helmet head'-type feeling for the consumer. Performance benefits achieved by the hairspray product pursuant to the present invention include excellent hair feel, particularly natural hair feel, and excellent non-stickiness of the hands and hair, good hold and shapeable hold. Furthermore, it has been surprisingly found that the selected polymers can be blended together in order to provide more natural hair feel or greater hairstyle hold. The hairstyling polymers can be blended into specific mixtures, for example 'soft' hairstyling polymers may be blended with 'hard' hairstyling polymers.

A particular benefit of the hairstyling polymers as described herein is the low tackiness on hands and/or hair achieved. Surprisingly the tackiness on hands and/or hair of the present invention is lower than achieved by conventional ethanol-based aerosol hairsprays. This is surprising because the hold provided by the present invention is comparable to conventional aerosol hairsprays.

Each of the features of the aerosol hairspray product, as well as other relevant components, are described in detail hereinafter.

According to the first aspect, the present invention relates to an aerosol hairspray product, wherein the product comprises less than 15% VOC by total weight of the hairstyling formulation and propellant. In an embodiment, the aerosol hairspray product has a maximum incremental reactivity (MIR) value of less than 1, or less than 0.8, or less than 0.7, or less than 0.4. The MIR value of an aerosol hairspray product can be calculated by multiplying the fraction by weight of each component of the hairspray product by its MIR value. MIR values of common components of hairspray products include: 2-aminomethyl propanol: 15.08; water: 0.00; acetone: 0.43; ethanol: 1.69; isopropanol: 0.71. More MIR values are listed below. For example, a product comprising 0.2% of 2-aminomethyl propanol and no other components with an MIR value above zero, would have an MIR value of 0.03. In an embodiment, the hairstyling formulation and propellant have a heat of combustion of from 5 kJ/kg to 20 kJ/kg and/or the product is non-flammable.

The surface tension and viscosity of the hairstyling formulation can be important because following spraying, the ejected composition forms droplets, which land on the hair. The ejected composition should then spread out along each individual hair fibre in order to form a thin layer of coating on the hair, which dries quickly and also forms welds with other similarly coated hair fibres. In an embodiment, the surface tension, measured according to standard test ISO 304 at 20°C, of the hairstyling formulation is from 20 mN/m to 50 mN/m, or from 20 mN/m to 40 mN/m, or from 28 mN/m to 40 mN/m, or from 30 mN/m to 40 mN/m. ISO 304 is a standard test method for measuring surface tension of pure liquids or solutions.

In an embodiment, the kinematic viscosity, measured according to standard test DIN EN ISO 3104, of the hairstyling formulation is from 1 $mm^2$/s to 25 $mm^2$/s, or from 1 $mm^2$/s to 15 $mm^2$/s, or from 2 $mm^2$/s to 10 $mm^2$/s, or from 1 $mm^2$/s to 4 $mm^2$/s, or from 1.2 $mm^2$/s to 3 $mm^2$/s. DIN EN ISO 3104 is a standard test method for measuring kinematic viscosity of liquids. The kinematic viscosity can be important because when the hairstyling formulation is too viscous then the hairstyling formulation is too thick and cannot be sprayed and/or is clogging - inhomogeneous ejected formulation results e.g. irregular spray beam, "spitting" rather than spraying, and/or ejection of lumps. This can be especially important when a compressed gas propellant is utilised because the propellant is in gaseous form and hence cannot function as a co-solvent.

The median droplet size of the ejected composition is from 10 micron to 80 micron, or from 15 micron to 60 micron, or from 15 micron to 50 micron, or from 20 micron to 35 micron. Droplets smaller than 10 micron are not suitable for the present invention due to safety concerns - the droplets may enter the lungs and cause health problems. Droplets larger than 100 micron are too large and consequently unsuitable. In an embodiment, the droplet size is not greater than 80 micron. Hairspray products which are pump sprays normally have a droplet size which is too large and are hence unsuitable. The aerosol hairspray product is not a pump spray.

Droplet size is measured using a technique based on laser diffraction. Scattered light is focused by a focusing lens in a Fourier arrangement and picked up by the detector array. The angle at which a particle/droplet diffracts light is inversely proportional to its size. The detector array is made up of over 30 individual detectors, each of which collects the light scattered by a particular range of angles. The scattering pattern from the spray is captured, which is what is measured. Measuring the angle of diffraction determines the size of the particle/droplet. A Malvern Spraytec EPCS 4.0 is used with a 450 mm lens type, serial number 237. Software: RT Sizer 5.0. Test duration: 4000 ms. Data acquisition rate: 200 Hz. Minimum droplet size able to be measured: 0.8 micron. Maximum droplet size able to be measured: 300 micron. Distance between nozzle and laser beam: 140 mm.

The ejection flow of the hairspray product is from 0.10 g/sec to 0.40 g/sec, or from 0.20 g/sec to 0.35 g/sec, or from 0.20 g/sec to 0.30 g/sec, or from 0.20 g/sec to 0.25 g/sec. If the ejection flow is greater than 0.45 g/sec, then the on-hair drying time will be too long for consumer satisfaction. Ejection flow can typically be adjusted by altering the pressure inside the container (increased pressure correlates with faster ejection flow) and/or the diameter opening in the spraying device and/or orifices in the actuator (lower diameter correlates with slower ejection flow).

The on-hair drying time of the ejected composition may be from 0.5 min to 7 min, or from 1 min to 5 min, or from 1 min to 2 min.

The hairstyling formulation comprises from 0.01% to 20%, or from 0.01% to 16%, or from 0.01% to 10%, or from 1% to 8%, or from 2% to 6% of a hairstyling polymer, by total weight of the hairstyling formulation and propellant.

The hairstyling polymer is selected from the group consisting of: acrylates copolymers of two or more monomers of (meth)acrylic acid or one of their simple esters; octylacrylamide/acrylate/butylaminoethyl methacrylate copolymers; acrylates/hydroxyesters acrylates copolymers of butyl acrylate, methyl methacrylate, methacrylic acid, ethyl acrylate and hydroxyethyl methacrylate; polyurethane-14/AMP-acrylates copolymer blend; and mixtures thereof. Balance® CR from Akzo Nobel is an acrylates copolymer of two or more monomers of (meth)acrylic acid or one of their simple esters. Amphomer® is an octylacrylamide/acrylate/butylaminoethyl methacrylate copolymer. Acudyne™ 1000 is an acrylates/hydroxyesters acrylates copolymer of butyl acrylate, methyl methacrylate, methacrylic acid, ethyl acrylate and hydroxyethyl methacrylate. DynamX® H2O from Akzo Nobel is blend of an acrylates copolymer and a polyurethane polymer i.e. polyurethane-14/AMP-acrylates copolymer blend. Balance® CR from Akzo Nobel is an acrylates copolymer of two or more monomers of (meth)acrylic acid or one of their simple esters. In an embodiment, the hairstyling polymer is selected from the group consisting of: acrylates copolymers of two or more monomers of (meth)acrylic acid or one of their simple esters; acrylates/hydroxyesters acrylates copolymers of butyl acrylate, methyl methacrylate, methacrylic acid, ethyl acrylate and hydroxyethyl methacrylate; polyurethane-14/AMP-acrylates polymer blend; and mixtures thereof.

In an embodiment, the hairstyling formulation comprises from 3% to 20% of a sole hairstyling polymer, wherein the sole hairstyling polymer is selected from the group consisting of: acrylates copolymers of two or more monomers of (meth)acrylic acid or one of their simple esters; or acrylates/hydroxyesters acrylates copolymers of butyl acrylate, methyl methacrylate, methacrylic acid, ethyl acrylate and hydroxyethyl methacrylate; or octylacrylamide/acrylate/butylaminoethyl methacrylate copolymers. As used herein "sole hairstyling polymer" means that the hairstyling formulation comprises only one type of hairstyling polymer and other hairstyling polymers are not present, wherein the other hairstyling polymers that do not fall within the definition provided for the sole hairstyling polymer.

In an embodiment, the hairstyling formulation comprises a mixture of hairstyling polymers. The mixture may comprise a hard hairstyling polymer and a soft hairstyling polymer. As used herein "hard hairstyling polymer" is a hairstyling polymer which provides excellent hairstyle hold and this hairstyle hold is more pronounced as the concentration of the hard hairstyling polymer in the hairstyling formulation increases. However, high concentrations of hard hairstyling polymer typically have negative effect on the hair feel i.e. consumers find the palpable feel of the hair unacceptable e.g. rough. As used herein "soft hairstyling polymer" is a hairstyling polymer which provides excellent i.e. natural hair feel, particularly soft and/or smooth hair feel, but typically the hairstyle hold provided is limited.

In an embodiment, the hairstyling formulation comprises from 3% to 20% of a sole hairstyling polymer, wherein the sole hairstyling polymer is a hard hairstyling polymer.

The softness and hardness of the hairstyling polymer depends on the M.Wt. and the glass transition temperature of the hairstyling polymer, and also the chemistry of the hairstyling polymer i.e. the chemistry of the monomers.

In an embodiment, the hard hairstyling polymer has a glass transition temperature of greater than or equal to 10°C and the soft hairstyling polymer has a glass transition temperature of less than 10°C. "Glass transition temperature" or "$T_g$", as used herein, means the lowest temperature at which a polymer can be considered flowable, which means the polymer chains can slide past each other when a force is applied. The $T_g$ as used herein may be measured according to DIN EN 61 006.

In an embodiment, the M.Wt. of the hairstyling polymer(s) is from 10 thousand g/mol to 200 thousand g/mol, or from 20 thousand g/mol to 150 thousand g/mol. In an embodiment, the hard hairstyling polymer has M.Wt. of from 90 thousand g/mol to 200 thousand g/mol. In an embodiment, the soft hairstyling polymer has M.Wt. of from 10 thousand g/mol to 90 thousand g/mol.

The hairstyle hold provided by increasing amounts of the soft hairstyling polymer in the hairstyling formulation increases, but then plateaus. In other words, the soft hairstyling polymer has a maximum hairstyle hold that it can provide. Consequently, it can be advantageous to provide a mixture of a hard hairstyling polymer and a soft hairstyling polymer.

In an embodiment, the hard hairstyling polymer is selected from the group consisting of: acrylates copolymers of two or more monomers of (meth)acrylic acid or one of their simple esters; octylacrylamide/acrylate/butylaminoethyl methacrylate copolymers; acrylates/hydroxyesters acrylates copolymers of butyl acrylate, methyl methacrylate, methacrylic acid, ethyl acrylate and hydroxyethyl methacrylate; and mixtures thereof. In an embodiment, the soft hairstyling polymer is selected from the group consisting of: a polyurethane-14/AMP-acrylates polymer blend; latex hairstyling polymers; polyesters; and mixtures thereof. In an embodiment, the soft hairstyling polymer is a polyurethane-14/AMP-acrylates polymer blend or a latex hairstyling polymer. In an embodiment, the hairstyling formulation comprises an additional soft hairstyling polymer, wherein the additional soft hairstyling polymer is selected from the group consisting of: PVP (polyvinylpyrrolidone) polymers; PVP-VA-copolymers (vinylpyrrolidone/vinylacetate copolymers); polyesters; and mixtures thereof.

In an embodiment, the hairstyling formulation comprises a mixture of: a soft hairstyling polymer being a polyurethane-14/AMP-acrylates polymer blend or a latex hairstyling polymer; and a hard hairstyling polymer selected from the group consisting of: acrylates copolymers of two or more monomers of (meth)acrylic acid or one of their simple esters; octylacrylamide/acrylate/butylaminoethyl methacrylate copolymers; and acrylates/hydroxyesters acrylates copolymers of butyl acrylate, methyl methacrylate, methacrylic acid, ethyl acrylate and hydroxyethyl methacrylate. In an embodiment, the mixture comprises polyester-5 and an acrylates copolymers of two or more monomers of (meth)acrylic acid or one of their simple esters. In an embodiment, the mixture comprises at least 2, or at least 3, different hairstyling polymers. In example of a polyester-5 polymer is AQ® 48 Ultra Polymer from Eastman Chemical Company.

In an embodiment, the weight ratio of hard hairstyling polymer to soft hairstyling polymer (hard:soft) in the mixture is from 10:1 to 1:10, or from 10:1 to 1:2.

In an embodiment, the hairstyling formulation further comprises a panthenol compound. In an embodiment, the panthenol compound is selected from the group consisting of: panthenol, a pantothenic acid derivative, and mixtures thereof. In an embodiment, the panthenol compound is selected from the group consisting of: D-panthenol ([R]-2,4-dihydroxy-N-[3-hydroxypropyl)]-3,3-dimethylbutamide), D/L-panthenol, pantothenic acids and their salts, panthenyl triacetate, royal jelly, panthetine, pantotheine, panthenyl ethyl ether, pangamic acid, pantoyl lactose, Vitamin B complex, and mixtures thereof. In an embodiment, the hairstyling formulation comprises a mixture comprising a hard hairstyling polymer, panthenol, and optionally a soft hairstyling polymer. In an embodiment, the hairstyling formulation comprises panthenol. The panthenol compound is able to have a 'softening' effect on the hard hairstyling polymer. The hairstyling formulation may comprise from 0.1% to 0.6%, or from 0.1% to 0.3%, of a panthenol compound by total weight of the hairstyling formulation and the propellant. The weight ratio of hard hairstyling polymer to panthenol compound maybe from 100:6 to 100:1, or from 100:4 to 100:20. In an embodiment, the panthenol is either D-panthenol or D/L-panthenol.

In an embodiment, the hairstyling polymer is a water-compatible hairstyling polymer, alternatively a water-soluble hairstyling polymer. In an embodiment, the hairstyling formulation is substantially free from a water-incompatible hairstyling polymer. Balance® CR, Amphomer®, Acudyne™ 1000, DynamX® H2O from Akzo Nobel are water-compatible.

In an embodiment, the hairstyling formulation comprises a latex hairstyling polymer. In an embodiment, the hairstyling polymer is a polyurethane polymer. In an embodiment, the polyurethane polymer is Polyurethane-48. Baycusan® C 1008 is a Polyurethane-48, which is an aqueous polyurethane dispersion. In an embodiment, the product comprises less than 0.5% of a cationic surfactant by total weight of the hairstyling formulation and propellant. In an embodiment, the hairstyling formulation comprises a polyurethane polymer and the hairstyling formulation is substantially free of a cationic surfactant. In an embodiment, the sole hairstyling polymer is neither a latex hairstyling polymer nor a polyurethane polymer.

The tackiness on hands and/or hair of the present invention is lower than achieved by conventional ethanol-based aerosol hairsprays. In a comparative embodiment, the product comprises from 20% to 50% VOC, by total weight of the hairstyling formulation and the propellant and the liquefied gas propellant is DME. The low tackiness on hands and/or hair benefit is also achieved for this embodiment.

Amphoteric polymers such as Amphomer® and anionic polymers such as Balance® CR are normally present in their neutralized or partially neutralized form. The hairstyling polymer is at least 60%, or at least 80% neutralized.

Suitable neutralisers include potassium hydroxide, sodium hydroxide, triisopropanolamine (TIPA), 2-aminobutanol, 2-aminomethyl propanol (AMP), aminoethylpropandiol, dimethyl stearamine (Armeen 18 D), sodium silicate, tetrahydroxypropyl ethylenediamine (Neutrol® TE), ammonia ($NH_3$), triethanolamine, trimethylamine (Tris Amino Ultra), aminomethylpropandiol (AMPD). In an embodiment, the neutralising agent is 2-aminobutanol, ammonia, or 2-aminomethyl propanol.

The hairstyling formulation may further comprise a surfactant. The hairstyling formulation may comprise 1% or less surfactant, or 0.6% or less, or 0.4% or less, or 0.3% or less, by total weight of the hairstyling formulation and propellant. In an embodiment, the surfactant is selected from the group consisting of cationic surfactants, non-ionic surfactants, anionic surfactants, and mixtures thereof. Cationic surfactants may be selected from the group consisting of cetrimonium chloride (e.g. Quartamin 60L-G from Kao; DEHYQUART A-CA /DETEX; ARQUAD 16-25 LO); cocamidopropyl hydroxysultaine (e.g. REWOTERIC AM CAS); cocamidopropyl betaine (e.g. TEGO BETAIN F 50); betaine; and mixtures thereof. Non-ionic surfactants maybe selected from the group consisting of: castor oil PEG-40 H (e.g. NEODOL 91-8); laureth-4 (e.g. DEHYDOL LS 4 DEO N); laureth-9; decyl glucoside (e.g. Plantacare 2000); polysorbate 20 (e.g. TWEEN 20 PHARMA from UNIQEMA); PEG-25 hydrogenated castor oil (e.g. SIMULSOL 1292 DF from SEPPIC); PEG-40 hydrogenated castor oil (e.g. CREMOPHOR CO 410 from BASF); PPG-1-PEG-9-laurylglycolether (e.g. Eumulgin L); siloxane polyalkyleneoxide copolymer (Silwet® L7604 from Momentive); and polydimethylsiloxane methylethoxylate (Silwet® L7600 from Momentive); and mixtures thereof. A suitable anionic surfactant is dioctyl sodium sulfosuccinate (DOSS or 1,4-dioctoxy-1,4-dioxobutane-2-sulfonic acid), an example of which is Aerosol OT-70 PG from Cytec. In an embodiment, the surfactant is selected from the group consisting of: castor oil PEG-40 H; cetrimonium chloride; laureth-4; laureth-9; decyl glucoside; cocamidopropyl hydroxysultaine; polysorbate 20; siloxane polyalkyleneoxide copolymer; dioctyl sodium sulfosuccinate; and mixtures thereof. In an embodiment, the surfactant is selected from the group consisting of: castor oil PEG-40 H; decyl glucoside; cocamidopropyl hydroxysultaine; polysorbate 20; siloxane polyalkyleneoxide copolymer; dioctyl sodium sulfosuccinate; and mixtures thereof. In an embodiment, the surfactant is selected from the group consisting of: siloxane polyalkyleneoxide copolymer; and dioctyl sodium sulfosuccinate; and mixtures thereof.

The hairstyling formulation comprises at least 50%, or from 50% to 99%, or from 60% to 99%, or from 70% to 99% water by total weight of the hairstyling formulation and propellant. When the product is substantially free of VOC, the hairstyling formulation may comprise from 90% to 99% water, by total weight of the hairstyling formulation and propellant.

The product comprises 2% or less alcohol by total weight of the hairstyling formulation and propellant. In an embodiment, the product comprises 1.8% or less, or 1.5% or less, or 1% or less, alcohol by total weight of the hairstyling formulation and propellant, or is substantially free of alcohol. In an embodiment, the hairstyling formulation is substantially free of ethanol and propanol. In an embodiment, the product comprises 5% or less, or 2% or less, or 1.8% or less, or 1.5% or less, or 1% or less, aliphatic alcohol by total weight of the hairstyling formulation and propellant. "Aliphatic alcohol" as used herein means an alcohol comprising no aromatic group.

The hairstyling formulation may comprise at least one preservative. The preservative may be present in an amount of less than 1.5%, or 0% to 1%, or 0.01% to 1% by total weight of the hairstyling formulation and propellant. Suitable preservatives include: phenoxyethanol (e.g. Euxyl® PE 9010), benzyl alcohol, propyleneglycol, PHMB (Poly-aminopropyl biguanide), Optiphen (Phenoxyethanol + caprylyl glycol) from ISP, Symtriol (1,2 octanediol and 1,2 hexanediol,Methylbenzyl alcohol) from Symrise, octylsalicylate, 1,3-bis(hydroxymethyl)-5,5-dimethylimidazolidine-2,4-dione (DMDM hydantoin; Nipaguard® DMDMH by Clariant), EDTA (Rexat), butylene glycol (Dekaben LMB), and parben types e.g. methylparaben (e.g. PHB-methyl ester from Schütz & Co., or SLI Chemicals, or Nipagin® M), propylparaben (PHB-propylester from Solvadis Specialties).

The hairstyling formulation may further comprise at least one perfume or fragrance. The aerosol hairspray product may comprise a maximum of 0.5% perfume or fragrance, or from 0% to 0.4%, or from 0.03% to 0.3%, by total weight of the hairstyling formulation and propellant.

The hairstyling formulation may further comprise vitamins and amino acids such as: water soluble vitamins such as vitamin B1, B2, B6, B12, C, pantothenic acid, pantothenyl ethyl ether, panthenol, biotin, and their derivatives, water soluble amino acids such as asparagine, alanine, indole, glutamic acid and their salts, water insoluble vitamins such as vitamin A, D, E, and their salts and/or derivatives, water insoluble amino acids such as tyrosine, tryptamine, viscosity modifiers, dyes, non-volatile solvents or diluents (water soluble and insoluble), pearlescent aids, foam boosters, additional surfactants or non-ionic cosurfactants, pediculocides, pH adjusting agents, perfumes, preservatives, chelants, proteins, skin active agents, sunscreens, UV absorbers, vitamins, niacinamide, caffeine and minoxidil. The product may comprise from 0.01% to 5% vitamins and/or amino acids, by total weight of the hairstyling formulation and propellant.

The aerosol hairspray product may further comprise pigment materials such as inorganic pigments, nitroso-, monoazo-, disazo-compounds, carotenoid, triphenyl methane, triaryl methane, chemicals of the quinoline, oxazine, azine, or anthraquinone type, as well as compounds which are indigoid, thionindigoid, quinacridone, phthalocianine, botanical, natural colors, and water-soluble components. The product may comprise from 0% to 5% pigment materials, by total weight of the hairstyling formulation and propellant. The formulation(s) described herein may also contain antimicrobial agents which are useful as cosmetic biocides. The product may comprise from 0.01% to 5% antimicrobial agents, by total weight of the hairstyling formulation and propellant.

The hairstyling formulation may have a pH of from 6 to 10, or from 7 to 10, or from 7 to 9.

The product comprises a propellant, which is selected from the group consisting of compressed gas propellants, liquefied gas propellants, and mixtures thereof.

The product may comprise a compressed gas propellant. The compressed gas propellants may be selected from the group consisting of air, nitrogen ($N_2$), nitrous oxide ($N_2O$), carbon dioxide ($CO_2$), and mixtures thereof. In an embodiment, the compressed gas propellant is air or nitrogen ($N_2$). In an embodiment, the compressed gas propellant is nitrogen ($N_2$). In an embodiment, the compressed gas propellant is not carbon dioxide ($CO_2$) - particularly when a hairstyling polymer may precipitate due to effect of the $CO_2$ in lowering the pH of the hairstyling formulation. Also $CO_2$ typically permeates through plastic material to a greater or lesser extent i.e. 0% permeation is typically unachievable. The term "air" is defined herein as a gas comprising approximately 78% nitrogen, 21% oxygen, and 1% of carbon dioxide, argon and other trace elements. Since the content of air can vary, in an embodiment the compressed gas propellant is nitrogen gas. As defined herein, the compressed gases $N_2$, $CO_2$, and $N_2O$ are all non-flammable. $N_2O$ has a GWP of 298. When the propellant is air, a maximum of 1 g is utilised as propellant.

CFCs are not suitable propellants for the present invention due to their ozone depleting properties. For example, CFC-12 has a GWP of 10,900. In an embodiment, the product has a GWP of 100 or less, or 50 or less, or 20 or less, or 10 or less, or 5 or less.

The product may comprise a liquified gas propellant. The liquefied gas propellant may be selected from the group consisting of dimethylether (DME), 1,1-difluoroethane (HFC-152a), 1,1,1,2-tetrafluoroethane (HFC-134a), pentane, *n*-butane, *iso*-butane, propane, *trans*-1,3,3,3-tetrafluoropropene (HFO-1234ze), and mixtures thereof. In an embodiment, the liquefied gas propellant is dimethylether (DME) or 1,1-difluoroethane (HFC-152a). In an embodiment, the preferred liquefied gas propellant is DME.

For the purposes of the present invention, all the liquefied gas propellants mentioned above are VOCs. Furthermore, as defined herein, *n*-butane is flammable (MIR = 1.15, GWP = 4); *iso*-butane is flammable (MIR = 1.23); propane is flammable (GWP = 3.3, MIR = 0.49); HFC-134a is non-flammable (GWP = 1400, MIR = 0.00); HFC-152a is flammable (GWP = 120, MIR = 0.02); HFO-1234ze is non-flammable (GWP = 6, MIR = 0.09); DME is flammable (GWP = 1, MIR = 0.81).

The product comprises less than 15% VOC, or is substantially free of VOC, by total weight of the hairstyling formulation and propellant. In another embodiment, the product comprises from 1% to less than 15% of a VOC, by total weight of the hairstyling formulation and propellant. In an embodiment where the propellant is a compressed gas propellant, the product comprises less than 15% of a VOC, by total weight of the hairstyling formulation and propellant.

The present invention comprises a container comprising a container wall which encloses a reservoir for storing a hairstyling formulation and a propellant. In an embodiment, the container wall comprises predominantly plastic material. In an embodiment, the container wall comprises at least 80% plastic material, or from 85% to 100%, by total weight of the container. The term "plastic" is defined herein as any polymeric material that is capable of being shaped or molded, with or without the application of heat, and then hardened into a desired form including, polymers, resins, and cellulose derivatives. Usually plastics are homo- or co-polymers of high M.Wt.. Cosmetic products contained in plastic containers are known. Plastic is a particularly advantageous material for containing cosmetic products because a greater variety of specific container forms may be created. The utilisation of plastic material(s) for a hairspray container provides an excellent means to deliver ease-of-use benefits to the consumer. For example, it is very easy to provide tactile advantages e.g. grip features, contours, and these tactile advantages can be designed with a high degree of specificity and accuracy. Furthermore, a plastic container can easily be moulded in one piece. Sealed plastic containers have a lower explosion potential than metal containers because, upon application of excessive temperature for example, due to the more elastic nature of plastic compared to metal, the plastic material may expand at a weak point in the container, e.g. where the container wall is thinner. Gradually and eventually the expansion at this weak point allows the high-pressured containers to escape via the formation of a hole. Furthermore, aesthetic benefits can also be realised more easily when a plastic container is used, for example, a transparent and/or translucent container material could be employed, and in addition to many other aesthetic benefits. From an environmental perspective, utilisation of a container comprising predominantly plastic material has sustainability benefits and results in a reduced carbon footprint than alternative container materials. Plastic is also more easily recycled than metal.

In an embodiment, the plastic material is selected from the group consisting of polyolefins, polyesters, polyamide, polyvinylchloride, acrylic, polycarbonates, polyethylene naphthalate (PEN), polyethylene terephthalate (PET), polystyrene, polyurethane, and mixtures thereof. In an embodiment, the plastic material is selected from the group consisting of polyethylene terephthalate (PET), polyethylene napththalate (PEN), and mixtures thereof. Polyethylene napththalate is available from Hoechst Trevira GmbH & Co. KG, under the trademark Polyclear®, including Polyclear® N10, Polyclear® N90 and Polyclear® N100.

The container may comprise polymers made from components derived from renewable sources i.e. non-petroleum sources. As used herein the term "sustainable polymer" means polymers made from components e.g. monomers, derived from renewable sources. Examples of renewable, non-petroleum sources include plants and microorganisms. The renewable, non-petroleum plants sources may include sugar cane, beets, corn, potatoes, citrus fruit, and woody plants. For example, ethanol can be produced from sugarcane. The ethanol may then be converted into ethylene, which can be polymerized to form polyethylene (PE). The monomers from which polypropylene (PP), polyester, and polyethylene terephthalate (PET) are synthesized, may also be derived from renewable sources. Sustainable polymers may be synthesized from monomers derived from starch and/or cellulose, or by modification of the polymer itself. Cellulosics are thermoplastic resins manufactured by the chemical modification of cellulose.

These sustainable plastic materials may be used as 100% of the plastic material utilized for the container wall, or blended into the petroleum-derived plastic material at varying levels in order to vary performance and/or for economic reasons. Certain materials derived from plant sources may be biodegradable. Sustainable polymers exhibiting biodegradability include aliphatic polyesters such as polylactic acid (PLA), polyglycolic acid (PGA), polybutylene succinate (PBS) and copolymers thereof, aliphatic-aromatic polyesters such as Ecoflex® from BASF and Biomax® from DuPont, polyhydroxyalkanoate (PHA) and copolymers thereof. Thermoplastic starch (TPS) materials are also biodegradable, as are cellulosics. The incorporation of biodegradable sustainable polymers may be at 100% of the utilized plastic material or in blends with other materials, in order to control the speed or degree of biodegradation, or for economic reasons. The speed and degree of biodegradation must be compatible with the purpose and features of the present invention. Ecoflex® from BASF, for example, is a biodegradable plastic material that biodegrades in soil or compost. It is stable on shelf for one year. It is particularly suitable for bags and films.

Recycled plastic material can also be re-ground. This post-consumer regrind resin may also be suitable for the present invention either when blended with other resins or used as 100% of the plastic material utilised. Re-ground polyethylene at certain densities (r-HDPE, r-LLDPE, r-LDPE), reground polypropylene (r-PP), and reground polyethylene terephthalate (r-PET) may be suitable.

Filler materials may be blended into the plastic material. The advantages of the incorporation of filler materials into plastic material include: adjustment of physical properties of the plastic, such as mechanical strength, density and cooling time, and also economic reasons. In an embodiment, the filler is selected from the group consisting of: starches, fibres from renewable sources such as hemp, flax, coconut, wood, paper, bamboo, and also inorganic materials such as calcium carbonate, mica, and talc. In addition, gas fillers such as high pressure gas, foaming agents or microspheres may be added to the plastic material.

Plastic materials can be defined by their glass transition temperature (Tg) and/or M.Wt.. When the container wall comprises at least 80% plastic material, or from 85% to 100% plastic material, by total weight of the container, the wall thickness of the container wall may also be important. In an embodiment, the plastic material is PET, wherein the glass transition temperature of from 70°C to 80°C, and wherein the wall thickness is from 0.5 mm to 3.2 mm. An example PET container comprises the following wall thicknesses: shoulder 0.65 mm; sidewall 0.50 mm; outside base 1.09 mm; base pushup 2.90 mm. The container may be moulded to create a specific ergonomic external form or contour, for example, hand-shaped contours. Said form facilitates effective and precise use of the hairspray product, for example by providing more grip or non-slip. Other tactile features may also be provided on the surface of the container, for example pimples. In an embodiment, the container is not cylindrical in shape. Furthermore the container may be provided with specific aesthetic features, such as colour combinations, and transparent or translucent portions. In an embodiment, at least 50% of the container wall is translucent, or transparent. When externally viewable, bag-on-valve systems are less favoured by consumers for aesthetic reasons.

In an embodiment, the container wall comprises predominantly metal material. In an embodiment, metal material is selected from the group consisting of aluminium, tin plated steel, and combinations thereof. In an embodiment, the container wall comprises at least 80%, or from 85% to 100% metal material, by total weight of the container. In an embodiment, the container wall comprises at least 80% metal material by total weight of the container, and wherein the metal material is selected from the group consisting of: aluminium, tin plated steel, and combinations thereof; and wherein the propellant is a liquefied gas propellant, and wherein the liquefied gas propellant is selected from the group consisting of DME, 1,1-difluoroethane, 1,1,1,2-tetrafluoroethane, pentane, *n*-butane, *iso*-butane, propane, *trans*-1,3,3,3-tetrafluoropropene, and mixtures thereof; or DME, 1,1-difluoroethane, and mixtures thereof. In an embodiment, the container wall comprises an inner surface, wherein the inner surface is coated with a corrosion inhibitor. In an embodiment, the corrosion inhibitor is a polyamide-imide laquer. A suitable corrosion inhibitor is HOBA 8460, supplied by HOBA Lacke und Farben GmbH.

In an embodiment, the propellant and hairstyling formulation may freely communicate with one another inside the reservoir. In an embodiment, the propellant and hairstyling formulation are stored in a single compartment. In an embodiment, the aerosol hairspray product does not comprise a bag-on-valve system, especially when a portion of the container wall is translucent, or transparent. In an embodiment, the reservoir comprises a plurality of compartments for storing the hairstyling formulation and the propellant. In an embodiment, the propellant and hairstyling formulation are not stored in separate compartments. In an embodiment, the reservoir does not comprise a plurality of compartments for storing the hairstyling formulation and the propellant.

The pressure inside the reservoir can be measured with a pressure gauge (GCAS #60001439). The pressure inside the reservoir may be from 1 bar to 16 bar at 50°C. When the propellant is a compressed gas, the pressure inside the container may be from 6 bar to 12 bar, or from 8 bar to 10 bar, or from 9 bar, at 50°C. When the propellant is a liquefied gas, the pressure inside the container may be from 1 bar to 7 bar, or from 3 bar to 5 bar, at 50°C. In an embodiment, the reservoir comprises a maximum volume of 220 ml of hairstyling formulation and propellant.

The product comprises a spraying device attached to the container for dispensing the hairstyling formulation from the reservoir of the container. The sealing valve and actuator may or may not be made from plastic material. Valve and actuators are, for example, available from Seaquist Closures (Freyung, Germany), Aptar, Precision and Coster (Switzerland). US3,819,090 relates to a valve cup device for pressurized dispensing containers comprising a one-piece molded plastic body. US5,199,615A relates to an aerosol dispenser. A suitable spraying device when the propellant is a liquefied gas propellant is as follows: valve: Precision; stem: 0.010 inch; restricted tail piece: 0.050 inch; vapour phase housing: 0.020 inch; actuator: Kosmos 0.016 inch Wirbel; diptube : capillar 0.060 inch. Suitable spraying devices include NAZ DR 5113" and NAZ DR 5118 both from Aptar. The spray properties can be altered by utilising a vapour housing hole in the stem, which can help to make a wet spray drier by using more gas from the vapour phase. In an embodiment where the propellant is a liquefied gas propellant, the spraying device comprises a vapour housing hole.

In an embodiment, the spraying device comprises:

(I) a spray nozzle (2) for atomizing the hairstyling formulation, said nozzle (2) comprising
a fluid chamber (40), preferably a ring chamber, for receiving the hairstyling formulation from the reservoir (122) of the container, at least one feeding channel (42) for feeding the hairstyling formulation from the fluid chamber (40) radially inward into a swirl chamber (44) and an outlet channel (18) with an entrance end (54) facing the swirl chamber (44) and an exit end (56) for atomizing the hairstyling formulation to the environment of the spray nozzle (2), wherein the outlet channel (18) tapers in the dispensing direction of the hairstyling formulation, and/or

(II)

(a) a spray nozzle (2) for atomizing the hairstyling formulation, said nozzle (2) defining an axial direction and having a longitudinal axis (L-L) therethrough;
(b) at least one discrete inlet port (92), said inlet port (92) having an associated inlet area, said inlet port (92) not circumscribing said longitudinal axis (L-L) and being radially offset therefrom;
(c) a flow area joining said inlet port (92) and said nozzle (2), said flow area comprising a surface of revolution said longitudinal axis (L-L), said surface of revolution convergently directing flow from said at least one inlet port (92) to said nozzle (2); wherein at least a portion of the surface of revolution circumscribing said longitudinal axis (L-L) is curvilinear.

The spray nozzle according to (I) comprises a fluid chamber for receiving the hairstyling formulation. It is preferred, if the fluid chamber is configured as a ring chamber, which is more preferably in fluid connection with a fluid storage chamber for storing the hairstyling formulation. Said ring chamber is most preferably connecting with the swirl chamber. The spray nozzle further comprises at least one feeding channel for feeding the hairstyling formulation from the fluid chamber radially inward into a swirl chamber. Usually, one feeding channel may be sufficient, however, it has been found out that two, preferably three, or more feeding channels effect a better production of swirl within the swirl chamber and consequently a more symmetrical spray pattern. It is further preferred, if the feeding channel tangentially leads into the swirl chamber. According to the invention, there is further provided an outlet channel. The outlet channel has an entrance end facing the swirl chamber and an exit end for discharging the hairstyling formulation to the environment of the spray nozzle. In order to provide a spray nozzle needing a relatively low pump pressure and which is applicable even if highly viscous or tough-flowing hairstyling formulations have to be dispensed, the outlet channel tapers in the flow direction of the hairstyling formulation. It has been found out that the decrease of pressure within the spray nozzle could be reduced by using the tapered outlet channel. Further, the outlet channel tapering in the flow direction has positive effects on the spray pattern.

The spraying device according to (II) may be constructed as a cup, the so called "helix cup". The helix cup has a funnel wall which is not frustro-conical. This geometry provides a flow area defined as a convergent surface of revolution having a curvilinear funnel wall. In other words: The helix cup comprises: an inlet and an outlet defining a longitudinal axis therebetween, a funnel wall extending from said inlet to said outlet, said inlet having an inlet area, and said outlet having an outlet area, said inlet area being greater than said outlet area, and at least one concave or convex portion between said inlet and said outlet.

According to an embodiment, the outlet channel of the spray nozzle according to (I) tapers steadily or/and a tapering portion of the outlet channel abuts the exit end or/and the tapering portion also abuts the entrance end.

Basically, the outlet channel may taper stepwise in the flow direction. However, it is preferred that the outlet channel tapers steadily, thereby achieving a further reduction of the necessary pump pressure and a further lowering of the decrease of pressure within the nozzle.

In principle, a tapering portion of the outlet channel may be provided anywhere along the outlet channel to at least partially achieve the advantages mentioned above. However, in a further preferred embodiment of the spray nozzle the outlet channel comprises a tapering portion within which the outlet channel tapers in the flow direction, said tapering portion abutting the exit end of the outlet channel. In other words, the end of the tapering portion forms the exit end of the outlet channel, so that the outlet channel tapers in the flow direction until it reaches the exit end of it. A further reduction of the necessary pump pressure and a further lowering of the decrease of pressure within the nozzle could be achieved hereby. Above this, the described modification improves the spray pattern.

In a further preferred embodiment of the spray nozzle the tapering portion does not only abut the exit end of the output channel, the tapering portion rather abuts the entrance end of the outlet channel as well. In other words, the one end of the tapering portion forms the exit end of the outlet channel while the other end of the tapering portion forms the entrance end of the outlet channel, so that the tapering portion runs form the one end of the outlet channel to the other end of the outlet channel. Thus, the whole outlet channel tapers in the flow direction. It has been found out that the spray nozzle could thereby further be improved with regard to the advantageous effects mentioned above.

Due to the production method of the spray nozzle, the edge surrounding the exit end of the outlet channel is rounded, so that it has a radius. Thus, according to another embodiment, an edge surrounding the exit end of the spray nozzle according to (I) has a radius being smaller than 0.03 mm, preferably smaller than 0.02 mm, or/and the exit end has a maximum diameter between 0.1 and 0.2 mm, more preferably between 0.12 and 0.18 mm. It has been found out that the spray nozzle could further be improved with regard to the advantageous effects by limiting the radius of the edge accordingly.

According to another embodiment, the degree of tapering is constant in the flow direction, preferably wherein at least a tapering portion of the outlet channel or the whole outlet channel having the form of a truncated cone or a truncated pyramid.

In a further advantageous embodiment the degree of tapering increases in the flow direction. It has been found out that the hairspray product could further be improved with regard to the advantageous effects mentioned above by increasing the degree of tapering in the flow direction. It especially has a positive effect on the spray pattern and the spray angle without having a negative influence on the pressure drop within the nozzle and the pump pressure necessary to dispense the hairstyling formulation. Very good results have been achieved by providing an inner face of the outlet channel which is curved in the flow direction, this modification being further preferred in this embodiment. In this connection it is further preferred if at least a tapering portion of the outlet channel or the whole outlet channel more has the form of a spherical layer or a truncated paraboloid of revolution.

In a further and alternative, respectively, embodiment of the hairspray product the degree of tapering decreases in the flow direction. Again, it has been figured out that the hairspray product could further be improved with regard to the advantageous effects mentioned above by decreasing the degree of tapering in the flow direction. As in the embodiment described hereinbefore, it especially has a positive effect on the spray pattern and the spray angle without a negative influence on the pressure drop within the nozzle and the pump pressure necessary to dispense the hairstyling formulation. The inner face of the outlet channel is preferably curved in the flow direction. More preferably at least a tapering portion of the outlet channel or the whole outlet channel has the form of a truncated hyperboloid of revolution.

According to another embodiment, the inner face of the outlet channel of the spray nozzle according to (I) includes an angle, said angle preferably varying between 40° and 160°, more preferably between 80° and 120°, most preferably between 90° and 115° in order to achieve a positive effect on the spray pattern and the minimum pump pressure necessary to dispense the hairstyling formulation.

According to another embodiment, the feeding channel of the spray nozzle according to (I) comprises a first section and a second section following the first section in the flow direction and abutting the swirl chamber. In order to keep up the pressure within the nozzle and to avoid a pressure drop, respectively, the width of the first section preferably decreases in the flow direction. In this embodiment, the width of the second section is preferably constant or decreases to a lesser extent than the width of the first section in the flow direction. In this embodiment, it is further preferred if the side walls of the first section include an angle, said angle being subdivided into a first angle and a second angle by a centerline of the second section, the maximum difference between the first angle and the second angle being 10°, more preferably 5° or 1°. In the ideal case the first angle corresponds to the second angle. It has been found out that the positive effects mentioned before, especially keeping up the pressure within the nozzle and creating of the swirl within the swirl chamber, could further be enhanced.

In order to further enhance the positive effects of the invention as mentioned before, in a further advantageous embodiment the length of the second section in the flow direction is equal to or smaller than the width of the second section.

In order to avoid the pressure drop within the nozzle, thereby reducing the minimum pump pressure of the spray nozzle, in a further advantageous embodiment the height of the first or/and second section is decreasing in the flow direction. In this case it is also preferred if the degree of height reduction is constant, decreasing or increasing in the flow direction. If the degree of height reduction is decreasing or increasing it is further preferred if the bottom or the top of the second section is curved.

According to another embodiment, the spray nozzle according to (I) is assembled from a first element comprising protrusions for forming the side walls of the feeding channels and grooves between the protrusions and a second element being supported on the protrusions and covering the grooves in order to form the feeding channels, the protrusions comprising a support surface for supporting the second element.

Usually the second element being supported on the protrusions and covering the grooves in order to form the feeding channels is part of the sprayer for which the nozzle is intended to be used.

In order to further facilitate assembly the second element is a separated part which can be jammed or welded into the first element. In this preferred embodiment, to facilitate assembly, the second element can be jammed or welded into the first element and is molded together with the first element in one single part and connected by a flexible connecting piece.

Due to the production method of the spray nozzle, the transition region between the support surface of the protrusions and the surface of the protrusions facing the feeding channel may be rounded, so that it has a radius. In a further preferred embodiment of the spray nozzle the ratio of the radius between the support surface of the protrusion and the surface of the protrusion facing the feeding channel to the width of the feeding channel is equal to or less than 1/3, more preferably equal to or less than 1/4, most preferably equal to or less than 1/5, in order to achieve a compact cross-sectional form and to reduce the pressure drop within the nozzle.

According to another embodiment, the bottom is conical in the longitudinal direction forming with the second part a contact area which is defined by the penetration of the second part during the assembly, which generate pretension between the two parts due slightly bending the bottom of the nozzle in longitudinal direction.

In order to facilitate the assembly of the spray nozzle and to ensure a tight support of the second element on the first element, in a further preferred embodiment one of the first and second element comprises an elastic portion, said elastic portion being elastically deformed by the other element when the elements are assembled. In other words, one of the first and second elements is elastically pressed against the other of the first and second elements when the elements are assembled. This pretension further avoids separation of the first and second element if high pressures occur within the spray nozzle.

In a further advantageous embodiment the protrusions or/and the section of the first element carrying the protrusions form the elastic portion. The section of the first element carrying the protrusions is preferably a bottom of a cup-like first element being fixed to a surrounding wall of the cup-like first element. In this case, it is further preferred if the bottom is curved or convex towards the second element, before the first and second element are assembled. Said bottom may for example be more elastic than the surrounding wall.

In order to achieve an easily adjustable and flexible spray nozzle with regard to the pump pressure, the volumetric flow, the spray pattern, the spray angle or the like, in a further preferred embodiment the assembled first and second element are movable relative to each other into different relative positions thereby elastically changing the form, dimensions or/and justification of the feeding channels or/and the swirl chamber. It is further preferred, if the elements are lockable in their different relative positions, so that the change in pump pressure, volumetric flow, spray pattern, spray angle or the like could be kept up without the need to hold both elements in their relative position manually.

In order to further facilitate the production of the spray nozzle and the handling of the same during the assembling, in a further advantageous embodiment the first element and the second element are connected via a flexible connecting piece. Thus the two elements may be moved relative to each other during the assembling without the risk that one or the other element gets lost. The flexible connecting piece it preferably formed by a strip. It is further preferred, if the connecting piece is integrally formed or moulded with the first and second element or at least a part of the first and second element in order to facilitate the production of the spray nozzle.

In a further advantageous embodiment an outlet layer with a first hole, a channel layer with a second hole and slots and an inlet layer with holes are provided, said layers being sandwiched such that the first hole forms the outlet channel, the second hole forms the swirl chamber, the slots form the feeding channels and the holes in the inlet layer form inlet holes for feeding the hairstyling formulation from the fluid chamber into the feeding channels. The layers could for example be integrally formed, e. g. by galvanisation.

In order to allow a quick cleaning and prototyping of the spray nozzle, in a further preferred embodiment the layers are separable from each other or/and each of the layer is replaceable. Above this, this modification allows to produce a plurality of combinations of layers out of a few prefabricated layers without the need to provide the same number of single layers. The separable or/and replaceable layers could for example be provided in the form of thin discs.

In order to provide a spray nozzle being flexible with regard to the pump pressure, the volumetric flow, the spray pattern, the spray angle or the like, in a further preferred embodiment of the spray nozzle there is provided an overlapping area between the inlet holes and the feeding channels, in order to feed the hairstyling formulation through the inlet holes into the feeding channels. The size of the overlapping area or/and the distance between the overlapping area and the swirl chamber is preferably adjustable. By changing the size of the overlapping area or/and the distance between the overlapping area and the swirl chamber, the spray pattern or the like could be easily changed. In order to facilitate the handling of the spray nozzle, the inlet layer and the channel layer are more preferably moveable, most preferably rotatable, relative to each other in order to adjust the size of the overlapping area or/and the distance between the overlapping area and the swirl chamber. As alternative in order to further facilitate the handling of the spray nozzle, the inlet layer and the channel layer are more preferably moveable, by snapping them, rotating them on their axis to adjust the size of the overlapping area or shifting them by lateral movement to exchange a layer.

According to another embodiment, the spraying device according to (II) further comprises at least one inlet groove, said inlet groove having a first end intercepting an annular chamber disposed upstream of said inlet port, said inlet groove connecting said annular chamber and said inlet port.

According to another embodiment, the spraying device according to (II) comprises a plurality of inlet grooves, each said inlet groove connecting said annular chamber to said surface of revolution through a respective inlet port.

According to another embodiment, the spraying device according to (II) comprises four inlet grooves, said inlet grooves being equally circumferentially spaced the longitudinal axis.

According to another embodiment, the surface of revolution of the flow area of the spraying device according to (II) has at least a portion of which is concave relative to the longitudinal axis.

According to another embodiment, the surface of revolution of the flow area of the spraying device according to (II) has at least a portion of which is convex relative to the longitudinal axis.

According to another embodiment, the spraying device according to (II) comprises:

A spray nozzle through which hairstyling formulation may be sprayed, said nozzle defining an axial direction and having a longitudinal axis therethrough;

a groove extending from an inlet to an associated and discrete inlet port, said inlet port having an associated inlet area, said inlet port not circumscribing said longitudinal axis and being radially offset therefrom, said groove having an associated groove length taken parallel to said longitudinal axis;

a flow area joining said inlet port and said nozzle, said flow area comprising a surface of revolution said longitudinal axis, said surface of revolution convergently directing flow from said at least one inlet port to said a nozzle; said surface of revolution circumscribing said longitudinal axis, whereby said surface of revolution has an associated surface length taken parallel to said longitudinal axis, whereby said surface length is greater than said groove length.

According to another embodiment, the groove forms an angle of 5 to 12 degrees with respect to a plane perpendicular to the longitudinal axis.

According to another embodiment, the surface of revolution of the flow area further comprises a portion of constant cross section juxtaposed with the outlet.

According to another embodiment, the spraying device has an inlet and an outlet longitudinally spaced therefrom, and the flow area has at least one concave or convex portion between the inlet and the outlet.

According to another embodiment the funnel wall forms an inlet angle with respect to the longitudinal axis at the inlet, and the funnel wall forms an outlet angle with respect to the longitudinal axis at the outlet, the inlet angle being greater than the outlet angle.

According to another embodiment, the area of the funnel wall is at least 10%, and preferably at least 20 % less than the area of a comparable area of a frustrum of a right circular cone having the same inlet radius, outlet radius and cone length.

According to another embodiment, the Helix cup further comprises at least one flow diverter disposed on the funnel wall, the flow diverter imparting a spiral flow component to hairstyling formulation flowing from the inlet to the outlet, and preferably the at least one flow diverter comprises a plurality of grooves in the funnel wall.

According to another embodiment, the inlet has an inlet area and the outlet has an outlet area, at least one of said inlet and said outlet being nonround.

According to another embodiment, the ratio of the inlet area to the outlet area is at least 10:1.

In an embodiment, the product further comprises a variable spray-angle nozzle and/or variable resin flux nozzle.

In the second aspect, the invention relates to a method for styling hair comprising the steps of: (i) applying to hair an ejected composition, which is ejected by the product according to the present invention; (ii) drying the ejected composition on the hair. The method may also comprise a step preceding step (i) wherein a hairdo or hairstyle is created. The method may also comprise a step preceding step (ii) but after step (i) wherein a hairdo or hairstyle is created.

In the third aspect, the invention relates to the use of the product according to the present invention, for fixing and/or shaping a hairstyle. In an embodiment of the third aspect, the use comprises using the product according to the present invention for fixing a hairstyle following the creation of a hairstyle. Alternatively, the use comprises using the product according to the present invention for creating and shaping a hairstyle.

BRIEF DESCRIPTION OF THE DRAWINGS

Preferred embodiments of the present invention will now be described, by way of example only, with reference to the drawings in which

Fig. 1      shows a cross-sectional side-view of a first embodiment of the spray nozzle according to (I);
Fig. 2      shows a cross-sectional view along line A-A in Fig. 1;
Fig. 3      shows a cross-sectional view along line B-B in Fig. 2;
Fig. 4      shows the enlarged section C of Fig. 1;
Fig. 5      shows the enlarged section C of Fig. 1 with a first modification;
Fig. 6      shows the enlarged section C of Fig. 1 with a second modification;
Fig. 7      shows a schematic view of a second embodiment of the spray nozzle according to (I);
Fig. 8      shows a schematic view of a third embodiment of the spray nozzle according to (I).
Fig. 9      is a perspective view of an illustrative aerosol hairspray product;
Fig. 10     is a perspective view of the illustrative spray cap of Fig. 9;
Fig. 11     is a top plan view of the spray cap of Fig. 10;
Fig. 12     is a vertical sectional view of the spray cap of Fig. 10, taken along line F - F of Fig. 11;
Fig. 13     is an enlarged partial view of the indicated area of Fig. 12, showing the helix cup and backstop within the housing;
Fig. 14     is enlarged view of the helix cup of Fig. 12;
Fig. 15     is perspective view of an illustrative helix cup showing the inlet and having four channels;

Fig. 16   is perspective view of an illustrative helix cup showing the inlet and having three channels;

Fig. 17   is perspective view of an illustrative helix cup showing the inlet and having two channels;

Fig. 18   is an enlarged, fragmentary sectional view of the helix cup of Fig. 14;

Fig. 19   is a profile of the helix cup of Fig. 18, showing the inlet and taken in the direction of lines 5A - 5A in Fig. 14;

Fig. 20   is a perspective view of the flow path from the annular chamber to the nozzle outlet of the helix cup of Fig. 15;

Fig. 21   is a perspective view of the flow path from the annular chamber to the nozzle outlet of the helix cup of Fig. 15, showing the cutting plane formed by the backstop;

Fig. 22   is a perspective view of the ports of the flow path from the annular chamber into the helix cup of Fig. 15;

Fig. 23   is a vertical sectional view of an illustrative helix cup having grooves with an approximately 2 degree skew angle;

Fig. 24   is a vertical sectional view of an illustrative helix cup having grooves with an approximately 11.5 degree skew angle;

Fig. 25   is a broken vertical sectional view of alternative embodiments of a helix cup, the upper embodiment having a single groove, and a funnel wall with convex, concave and constant cross section portions, the lower embodiment having no groove and a funnel wall with two convex portions having a concave portion therebetween;

Fig. 26   is a vertical sectional view of an alternative embodiment of a cap having a more rigid backstop and the helix cup omitted for clarity; and

Fig. 27   is an enlarged partial view of the indicated area of Fig. 26, showing the backstop with a helix cup inserted in the housing.

## DETAILED DESCRIPTION OF THE DRAWINGS OF THE INVENTION

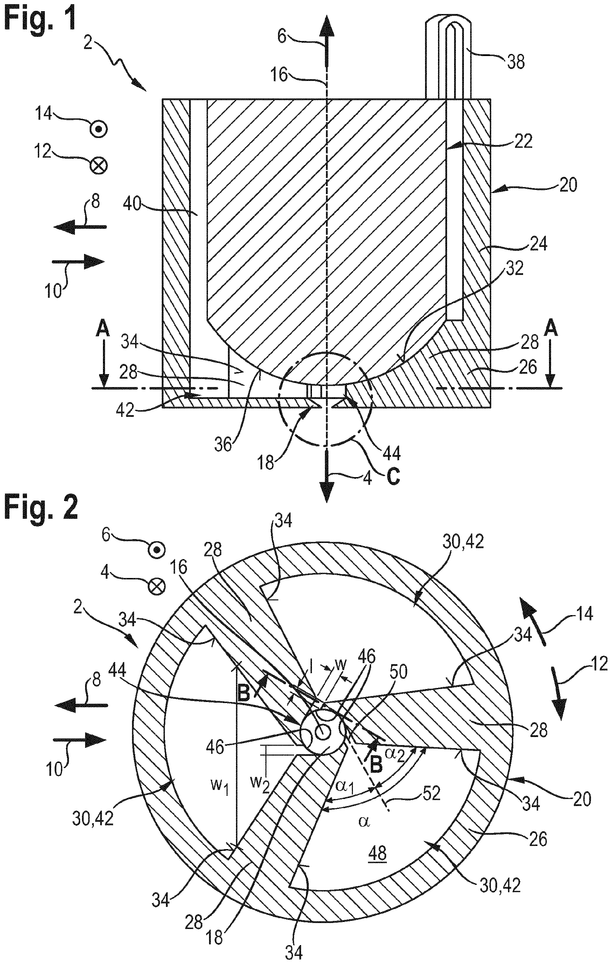

Fig. 1 to 4 show views of a first embodiment of the spray nozzle 2 for dispensing a hairstyling formulation. In the figures, the opposing longitudinal directions 4, 6, the opposing radial directions 8, 10 and the opposing circumferential directions 12, 14 of the spray nozzle 2 are indicated by corresponding arrows. The longitudinal axis 16 of the spray nozzle 2 extends in the longitudinal directions 4, 6, said longitudinal axis 16 further forming the centre axis of the outlet channel 18.

The spray nozzle 2 is assembled from a first element 20 and a second element 22. The first element 20 has a cup-like structure with a first section 24 extending in the circumferential directions 12, 14 and forming a surrounding wall and a second section 26 forming the bottom of the cup-like structure. The second section further comprises protrusions 28, said rib-like protrusions 28 extending in the longitudinal direction 6 and in the radial directions 8, 10. As can be best seen in Fig. 2, there are provided grooves 30 in the circumferential directions 12, 14 between the protrusions 28, said grooves being provided to form the feeding channels as will be described later. The protrusions 28 comprise an upper surface serving as a support surface 32 for supporting the second element 22, said support surface 32 facing the second element 22. Further, the protrusions 28 comprise side surfaces 34 facing the grooves 30 and feeding channels, respectively.

The second element 22 basically has a cylindrical form with a front face 36, said front face 36 bulging out in the longitudinal direction 4. In this embodiment, the front face 36 has a form of a spherical cap. The second element 22 is inserted into the cup-like first element 20, so that the front face 36 is supported on the support surfaces 32 of the protrusions 28. In this connection it should be mentioned, that the second element 22 may also be formed by a ball, which is pressed or clipped into the cup-like first element 20. Independent of the chosen form of the second element 22, it is preferred if the second element 22 could be snapped or clicked into its place within the first element 20, even if corresponding notches, snaps or the like for providing a form-fit or/and a force-fit are not shown in the figures.

The first element 20 and the second element 22 are connected via a flexible connecting piece 38, which - in this case - is formed by a strip. The connecting piece 28 is integrally formed or moulded with the second element 22 and at least the first section 24 of the first element 20. Even the second section 26 of the first element 20 may be integrally formed or moulded with the first section 24 of the first element 20 and consist of the same material. However, in this case the second section 26 has been subsequently fastened to the first section 24 since the second section 26 is made of a different material, as will be described hereinafter. Irrespective of the second section 26 being integrally formed with the first section 24 or not, the first element 20 comprises an elastic portion.

As already indicated above, the first element 20 is at least partially made of an elastic material being more elastic than the material of the second element 22. In this case, the second section 26 of the first element 20 with its protrusions 28 and its bottom section carrying said protrusions 28 is made of the elastic material, said elastic material being more elastic than the material of the second element 22 and more elastic than the material of the first section 24 of the first element 20. Thus, the afore-mentioned elastic portion of the first element 20 is essentially formed of the protrusions 28 and its bottom section carrying said protrusions 28. The elastic portion of the first element 20 is elastically deformed by the second element 22 when the elements 20, 22 are assembled. Even if the pre-assembled state is not shown, it is preferred if the bottom section carrying said protrusions 28 is curved or convex towards the second element 22 and in the longitudinal direction 6, respectively, before the first and second element 20, 22 are assembled.

The spray nozzle 2 is assembled by inserting the second element 22 into the cup-like first element 20 in the longitudinal direction 4 as shown in Fig. 1, thereby creating a fluid chamber 40, feeding channels 42 and a swirl chamber 44, while the outlet channel 18 is already provided in the second section 26 of the first element 20. The fluid chamber 40 is positioned in the radial directions 8, 10 between the first section 24 of the first element 20 and the second element 22, so that the fluid chamber 40 is formed as a ring chamber. The fluid chamber 40 receives the hairstyling formulation to be dispensed from a fluid storage chamber or container, which is not shown in the drawings. In the longitudinal direction 4 the fluid chamber 40 abuts the radial outer ends of the feeding channels 42, so that there is a fluid connection between the fluid chamber 40 and the feeding channels 42.

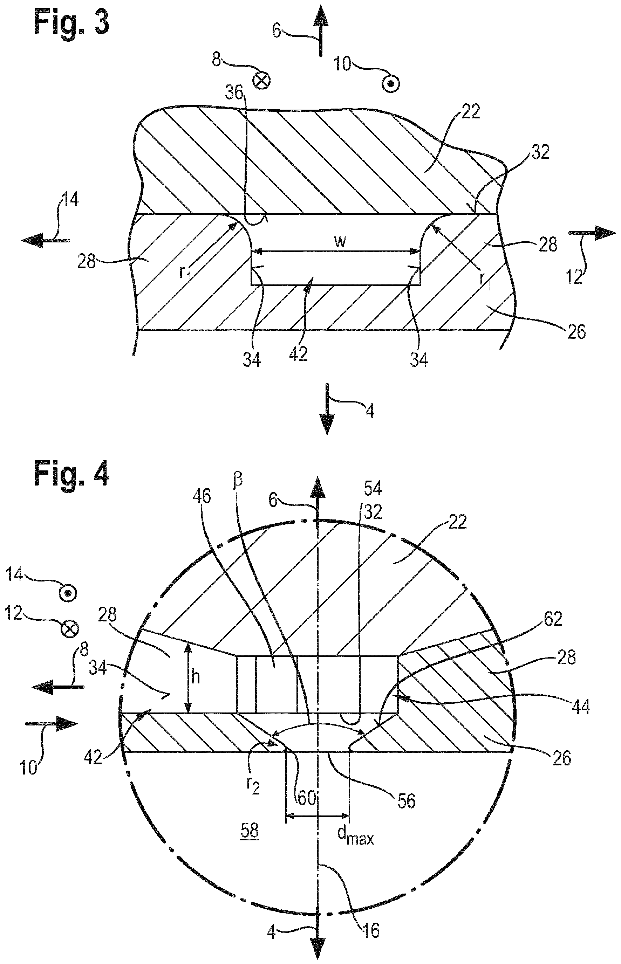

As can especially be seen in Fig. 2, the feeding channels 42 are extending radially inward to an exit end 46 of the feeding channels 42, where the feeding channels 42 abut the swirl chamber 44, so that the hairstyling formulation may be fed from the fluid chamber 40 via the feeding channels 42 into the swirl chamber 44. As shown in Fig. 3, the feeding channels 42 are limited in the circumferential directions 12, 14 by the side surfaces 34 of the protrusions 28, in the longitudinal direction 6 by the front face 36 of the second element 22, said second element 22 covering the grooves 30 to form the feeding channels 42, and in the longitudinal direction 4 by the bottom of the second section 26 carrying the protrusions 28.

The feeding channels 42 comprise a first section 48 abutting the fluid chamber 40 and a second section 50 following the first section 48 in the flow direction and radial direction 10, respectively. The second section 50 abuts the swirl chamber 44 with the exit end 46. As shown in Fig. 2, the width w1 of the first section 48 decreases in the flow direction and the radial direction 10, respectively. In contrast to this, the width w2 of the second section 50 is constant or decreases to a lesser extent than the first section 48 in the flow direction and radial direction 10, respectively.

The protrusions 28, which form the side walls of the first sections 48, include an angle $\alpha$. In Fig. 2, there is further indicated a centerline 52 of the second section 50 extending in the radial directions 8, 10. Said centerline 52 subdivides the angle $\alpha$ into a first angle $\alpha1$ and a second angle $\alpha2$. The maximum difference between the first angle $\alpha1$ and the second angle $\alpha2$ is 10°, more preferably 5° or 1°. Due to the bulged out front face 36 of the second element 22, at least the height h of the first section 48 of the feeding channels 42 decreases in the flow direction and the radial direction 10, respectively. Further, the length l of the second section 50 in the flow direction and the radial direction 10, respectively, is equal to or smaller than the width w2 of the second section 50.

As shown in Fig. 3, in the transition region between the support surfaces 32 and the side surfaces 34 the protrusions 28 comprise a radius r1. In order to have a compact cross-sectional form, the ratio of the radius r1 to the width w, e. g. w1 or w2, of the feeding channel 42 is equal to or less than 1/3, more preferably equal to or less than 1/4, most preferably equal to or less than 1/5.

Even if the first element 20 and the second element 22 are assembled, they are still movable relative to each other into different relative positions. In the shown embodiment, the elements 20 and 22 may be moved in the longitudinal direction 4, 6 relative to each other. By this relative movement the form, dimensions or/and justification of the feeding channels 42 or/and the swirl chamber 44 is changed by elastically deforming the protrusions 28 or/and the bottom of second section 26 of the first element 20, i.e. by elastically deforming the elastic portion of the first element 20. In other words, it is easy to change the behaviour of the spray nozzle 2. Further, there are provided means (not shown) for locking the elements 20, 22 in their different relative positions.

With reference to Fig. 4, the afore-mentioned outlet channel 18 in the second section 26 of the first element 20 comprises an entrance end 54 facing the swirl chamber 44 in the longitudinal direction 6 and an exit end 56 for discharging the hairstyling formulation to the environment 58 of the spray nozzle 2 and the sprayer, respectively, in the longitudinal direction 4. The outlet channel 18 tapers steadily in the flow direction and the longitudinal direction 4, respectively. Thus, the outlet channel 18 comprises at least one tapering portion. In the shown embodiment, the tapering portion abuts the exit end 56 as well as the entrance end 54 of the outlet channel 18, so that the whole outlet channel tapers in the flow direction. The edge 60 surrounding the exit end 56 has a radius r2. The radius r2 is smaller than 0.03 mm, preferably smaller than 0.02 mm. Further, the exit end 56 has a maximum diameter between 0.1 mm and 0.15 mm and more preferably a corresponding maximum cross-sectional area to achieve a suitable median droplet size of the ejected composition. A bigger geometry of the nozzle 2, which sprays a higher flow rate, may be advantageous in order to achieve smaller droplet/particle size at the same pressure and fluid characteristics and may allow the production of even smaller median droplet size than above. Above this, the outlet channel 18 has an inner face 62 surrounding the outlet channel 18 and limiting the same in the radial direction 8. The inner face 62 of the outlet channel 18 includes an angle $\beta$, said angle $\beta$ preferably varying between 40° and 160°, or between 80° and 120°, or between 90° and 115°.

As shown in Fig. 4, the degree of tapering of the outlet channel 18 is constant in the flow direction and the longitudinal direction 4, respectively. In the shown embodiment this is achieved by at least a tapering portion of the outlet channel 18 or the whole outlet channel 18 having the form of a truncated cone or a truncated pyramid. It has further been found out, that the pressure drop in the spray nozzle 2 could be reduced and a further reduction of the minimum pump pressure for dispensing the hairstyling formulation could be achieved by adjusting the ratio of the sum of the cross-sectional areas of the feeding channels 42 at their exit end 46 to the cross-sectional area of the exit end 56 of the output channel 18.

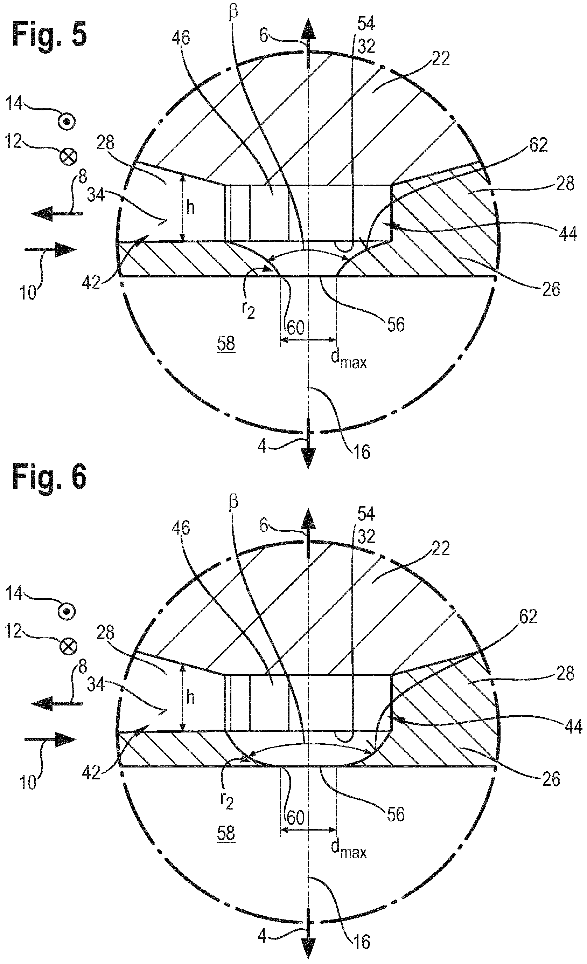

Fig. 5 shows the enlarged section C of Fig. 1 with a first modification. In the following only the differences will be described, the same reference signs will be used for similar or the same components and the above description of the first embodiment applies accordingly in this regard.

In contrast to the outlet channel 18 described with reference to Fig. 1 to 4, the degree of tapering of outlet channel 18 according to Fig. 5 decreases in the flow direction and the longitudinal direction 4, respectively. This is achieved by providing an inner face 62 of the outlet channel 18 being curved in the flow direction and the longitudinal direction 4, respectively. In the embodiment according to Fig. 5, at least the tapering portion of the outlet channel 18 or the whole outlet channel 18 has the form of a truncated hyperboloid of revolution.

Fig. 6 shows the enlarged section C of Fig. 1 with a second modification. In the following only the differences will be described, the same reference signs will be used for similar or the same components and the above description of the first embodiment applies accordingly in this regard.

In contrast to the outlet channel 18 described with reference to Fig. 1 to 4, the degree of tapering of outlet channel 18 according to Fig. 6 increases in the flow direction and the longitudinal direction 4, respectively. This is achieved by providing an inner face 62 of the outlet channel 18 being curved in the flow direction and the longitudinal direction 4, respectively. In the embodiment according to Fig. 6, at least a tapering portion of the outlet channel or the whole outlet channel has the form of a spherical layer or a truncated paraboloid of revolution.

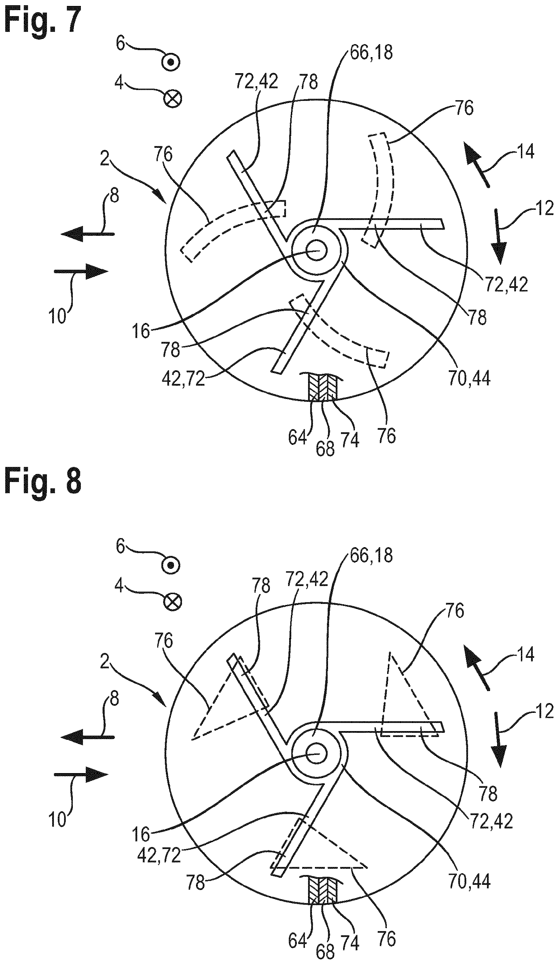

Fig. 7 shows a second embodiment of the spray nozzle 2. Since the second embodiment at least partially corresponds to the first embodiment according to Fig. 1 to 6, in the following only the differences will be described, the same reference signs will be used for similar or the same components and the above description of the first embodiment applies accordingly in this regard.

The spray nozzle 2 according to Fig. 7 comprises at least three layers, i.e. an outlet layer 64 with a first hole 66, a channel layer 68 with a second hole 70 and slots 72 and an inlet layer 74 with slot-like holes 76, said layers 64, 68 and 74 being sandwiched, while the inlet layer 74 is shown in a transparent manner in Fig. 7 to increase the intelligibility of the drawing. Being sandwiched this way, the first hole 66 forms the outlet channel 18, the second hole 70 forms the swirl chamber 44, the slots 72 form the feeding channels 42 and the holes 76 in the inlet layer form inlet holes for feeding the hairstyling formulation from the fluid chamber 40 into the feeding channels 42. In the shown embodiment, the layers 64, 68 and 74 are separable from each other and each of the layers 64, 68 and 74 could be replaced, so that the layers 64, 68 and 74 could also be regarded as separate discs with corresponding slots and holes.

As shown in Fig. 7, there is provided an overlapping area 78 between the inlet holes 76 and the feeding channels 42 when viewed in the longitudinal direction 4. The inlet layer 74 and the channel layer 68 are moveable - in this case rotatable around the longitudinal axis 16 - relative to each other, while the inlet holes 76 and the feeding channels 42 are formed such that, the distance between the overlapping area 78 and the swirl chamber 44 could be reduced by rotating the inlet layer 74 relative to the channel layer 68 in the circumferential direction 14 and could be enlarged by rotating the inlet layer 74 relative to the channel layer 68 in the circumferential direction 12. Thus, the distance between the overlapping area 78 and the swirl chamber 44 is adjustable.

Fig. 8 shows a third embodiment of the spray nozzle 2. Since the third embodiment at least partially corresponds to the second embodiment according to Fig. 7, in the following only the differences will be described, the same reference signs will be used for similar or the same components and the above description of the first and second embodiment applies accordingly in this regard.

In contrast to the second embodiment, the inlet holes 76 and the feeding channels 42 of the third embodiment are formed such that, the size of the overlapping area 78 could be reduced by rotating the inlet layer 74 relative to the channel layer 68 in the circumferential direction 12 and could be enlarged by rotating the inlet layer 74 relative to the channel layer 68 in the circumferential direction 14. Thus, the size of the overlapping area 78 is adjustable.

It should be mentioned that the principles of the second and third embodiment could also be advantageously combined in a single spray nozzle 2, so that the size of the overlapping area 78 as well as the distance between the overlapping area 78 and the swirl chamber 44 could be adjusted by a relative movement between the inlet layer 74 and the channel layer 68.

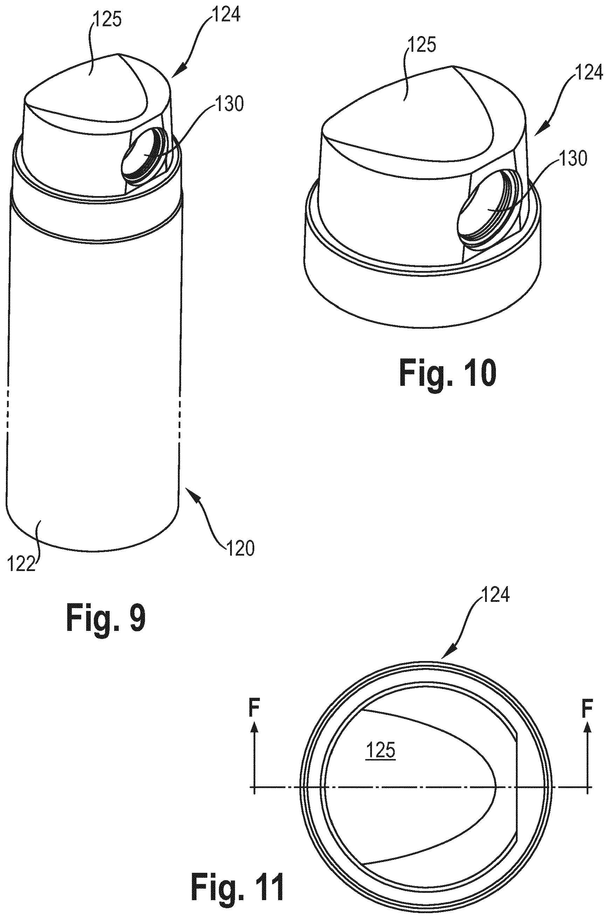

Referring to Figure 9, an aerosol hairspray product 120 is shown. The aerosol hairspray product 120 comprises a reservoir 122 for storing the hairstyling formulation and propellant. The product 120 may comprise a push button 125 valve system on or juxtaposed with the top. The product 120 may comprise a cap 124. The user manually depresses the push button 125, releasing the ejected composition under pressure from the reservoir 122 to be sprayed through the nozzle 2.

Referring to Figs. 10 and 11, the cap 124 further comprises a nozzle 2, through which the hairstyling formulation to be dispensed is atomized into small droplets. The nozzle 2 may be round, as shown, or have other cross sections. The nozzle 2 may be externally chamfered to increase the cone angle of the spray. A chamfer of 20 to 30 degrees has been found suitable. The droplets may be dispensed into the atmosphere or onto a target surface.

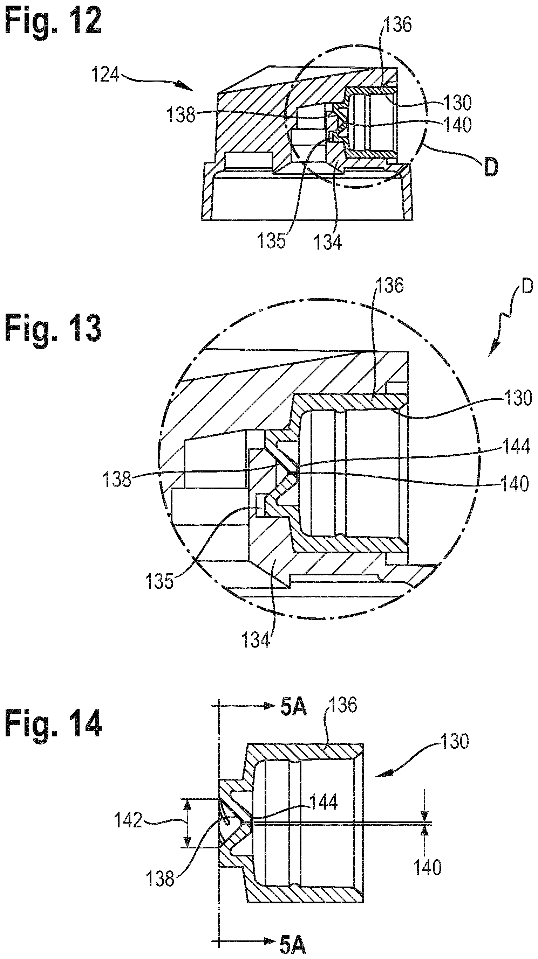

Referring to Figs. 12, 13 and 14, the invention comprises a helix cup 130. The helix cup 130 may be a discrete component insertable into a cap 124 of a spray system, as shown. Alternatively, the helix cup 130 may be integrally molded into the cap 124. The helix cup 130 may be injection molded from an acetal copolymer.

The helix cup 130 maybe inserted into the cap 124, and particularly into the housing 136 thereof. The housing 136 may have a backstop 134. The backstop 134 limits insertion of the helix cup 130 into the housing 136 of the cap 124. The backstop 134 further forms a cutting plane 184 with the helix cup 130.

Upon depressing the button 125 to initiate dispensing, the hairstyling formulation and propellant mixed therewith, is released from the reservoir 122 and flows through a valve. The hairstyling formulation enters a chamber 135 in the backstop 134 which chamber 135 is upstream of the cutting plane 184. The chamber 135 fills with the hairstyling formulation and the propellant to be dispensed. The chamber 135 may be annular in shape and circumscribe the axis of the nozzle 2.

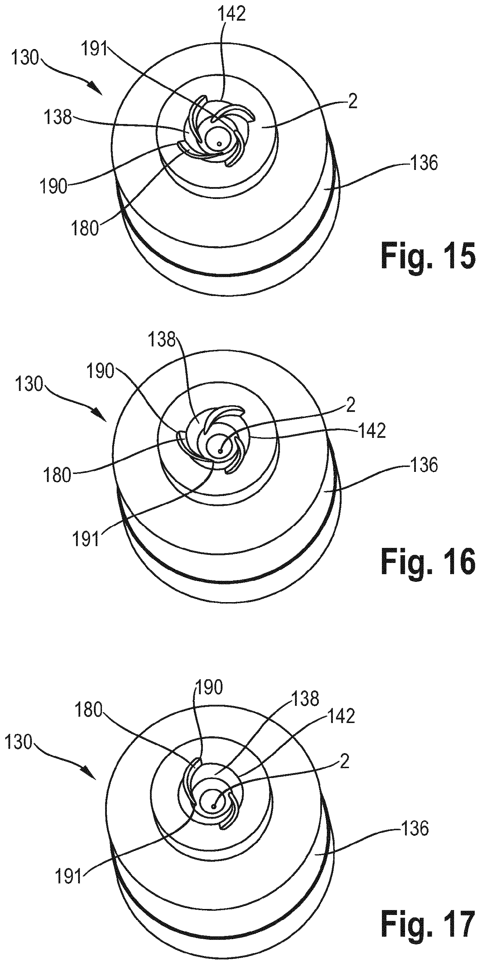

Referring to Figs. 15, 16, 17, the helix cup 130 may comprise a cylindrical housing 136. The housing 136 may have a longitudinal axis L-L therethrough. The helix cup 130 may have two longitudinally opposed ends, a first end with a funnel wall 138 and a generally open second end.

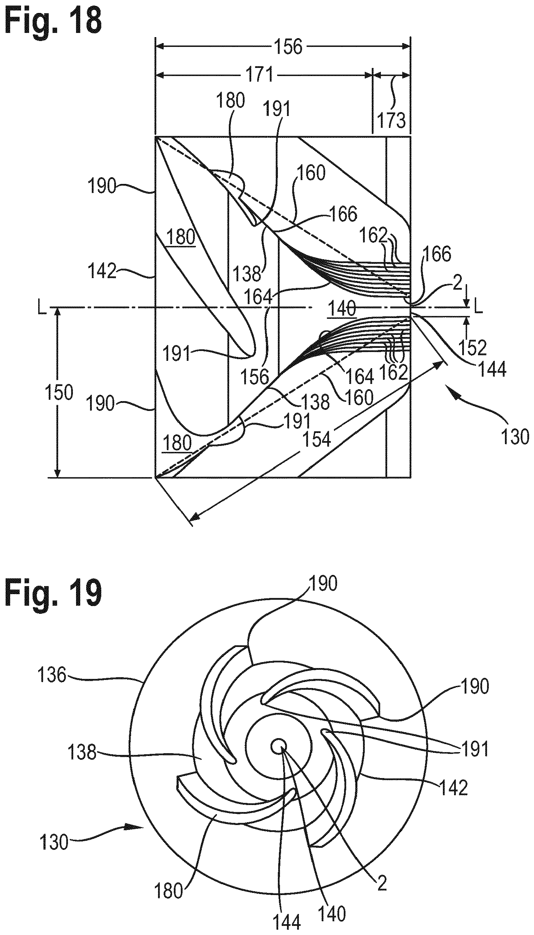

Referring to Figs. 18 and 19, an orifice may be disposed to provide a flow path through the funnel wall 138, and having an inlet and outlet 144. The outlet 144 may be the nozzle 2. The orifice may be centered in the helix cup 130, or may be eccentrically disposed. The orifice may be generally longitudinally oriented, and in a degenerate case parallel to the longitudinal axis L-L. The orifice may be of constant diameter or may taper in the axial direction. For the embodiments described herein, a constant orifice diameter of 0.13 mm to 0.18 mm may be suitable.

The funnel wall 138 has an inlet radius 150 at the first end and an outlet 144 radius corresponding to the nozzle 2 exit. The axial distance 156 between the inlet radius 150 and outlet 144 is parallel to the longitudinal axis L-L, and cone length 154 is the distance along the sidewall taken in the axial direction.

The prior art teaches a flow path having a frustrum of a right circular cone. This flow path provides a surface area given by:

$$(1) \quad \text{Area} = \Pi \text{ x cone length x (inlet radius + outlet radius)},$$

wherein the inlet radius 150 is greater than the outlet 144 radius, cone length 154 is the distance between the inlet and outlet 144 taken along the sidewall skewed relative to the longitudinal axis L-L, and II is the known constant of approximately 3.14.

For the helix cup 130, the area of the flow path may be at least 10%, 20%, 30%, 40%, 50%, 75% or 100% greater than the area of a comparable frustrum of a right circular cone having the same inlet radius 150, outlet radius 152 and cone length 154.

The subtended volume is given by:

$$(2) \quad \Pi/3 \text{ x h x [inlet radius }^2 + \text{ outlet radius }^2 + \text{ (inlet radius x outlet radius)]},$$

wherein h is the axial distance 156 between the inlet and outlet 144 taken parallel to the longitudinal axis L-L.

The frustrum flow path provides a convergent straight sidewall 160 shown in phantom, which would be predicted by one of ordinary skill to provide the least drag and flow resistance of all possible shapes. For example, in the book Sprays and Atomization by Lefebvre, page 116, it is specifically taught that straight, convergent sidewalls are known and used in the art.

For the helix cup 130, the sidewall 160 is not straight, but has a sidewall 160 with a curvilinear shape, i.e. the surface of revolution circumscribing the longitudinal axis L-L is curvilinear. Thus, the subtended volume of the flow path may be at least 10%, 20%, 30%, 40%, 50%, 75% or 100% greater than the subtended volume of a comparable frustrum of a right circular cone having the same inlet radius 150, outlet radius 152 and cone length 154. Likewise the helix cup 130, may have a subtended volume at least 10%, 20%, 30%, 40% or 50%, less than the subtended volume of a comparable frustrum of a cone.

Referring particularly to Fig. 18, it has been surprisingly found that improved results are achieved by having a longer flow path than is achievable with straight sidewalls. The longer flow path may be provided by having a funnel wall 138 which is concave, as shown. Fig. 18 further shows different hypothetical nozzle 2 diameters 162 usable with the funnel wall 138. The surface area of the funnel wall 138 will increase with greater nozzle 2 diameters 162, as illustrated.

As shown, the portion 164 of the funnel wall 138 juxtaposed with the orifice may be arcuate and the balance 166 of the funnel wall 138 may be straight. As used herein, straight refers to a line taken in the axial direction along the funnel wall 38 and may be thought of as the hypotenuse of a triangle disposed on the funnel wall 138, having one leg coincident the longitudinal axis L-L and having the other leg be a radius of the circle connected to the hypotenuse.

The funnel wall 138 of Fig. 18 may be conceptually divided into two portions, a first convergent portion 171 having variable flow area and a second straight portion 173 having constant flow area. The ratio of the axial length of the first area 171 to the second area 173 may be determined. For the embodiments described herein, the ratio of axial lengths of the first portion 171 to the second portion 173 may range from 1:3 to 3:1, from 1:2 to 2:1 or be approximately equal, providing a ratio of approximately 1:1. Furthermore, the ratio of the inlet area to the nozzle 2 area may be at least 1:1, 5:1, 7:1, 10:1 or 15:1.

Referring back to Figs. 15, 16, 17 the funnel wall 138 may have one or more grooves 180 therein, as shown. Alternatively, the funnel wall 138 may have one or more fins thereon. The grooves 180 or fins act to influence the flow direction. This influence imparts a circumferential directional component to the flow as it discharges through the orifice. The circumferential flow direction is superimposed with the longitudinally axial flow direction to provide a convergent helical, spiral flow path.

The grooves 180 may be equally or unequally circumferentially spaced the longitudinal axis L-L, may be of equal or unequal depth, equal or unequal length in the helical direction, equal or unequal width/taper. Figs. 15, 16, 17 show four, three and two axisymmetric grooves 180, respectively, although the invention is not so limited and may comprise more or fewer grooves 180 in symmetric and asymmetric dispositions, sizes, geometries, etc. The grooves 180 have a variable circumferential component, tapering towards the longitudinal axis L-L as the nozzle 2 is approached. To approach the nozzle 2, one of skill will recognize the grooves 180 also have an axial component.

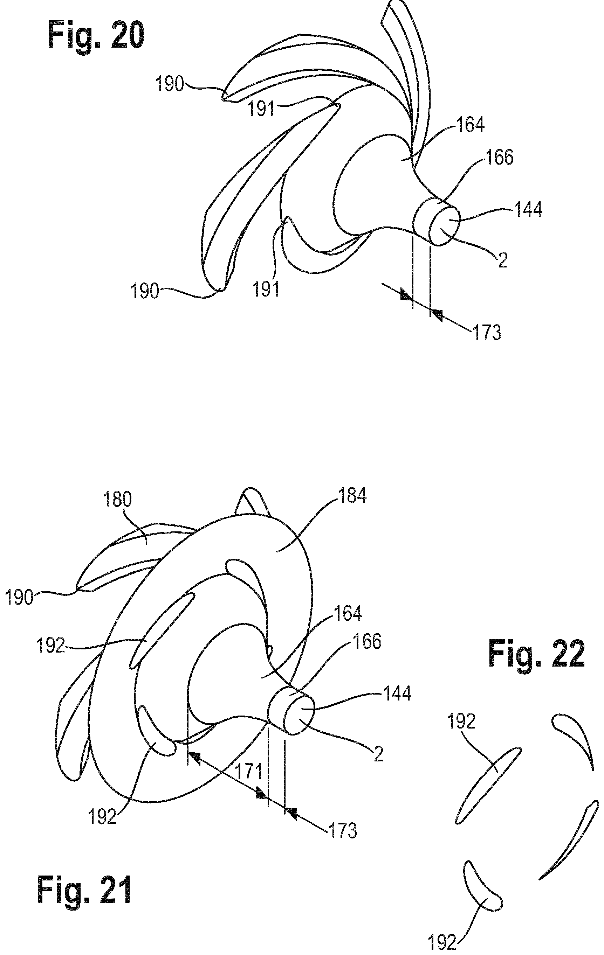

Referring to Figs. 20 - 21, the hairstyling formulation flow path is shown for the embodiment of Fig. 15 having four equally spaced and equally sized grooves 180. The flow enters the annular chamber 135 of the backstop 134, flows into each of the four grooves 180, passes the cutting plane 184 and enters the helix cup 130. The cutting plane 184 is a virtual plane which conceptually divides the flow between the grooves 180 and the convergent portion of the flow path 171.

Referring to Fig. 21, each groove 180 has a first end 190, which is the upstream end of the groove 180. The upstream end of the groove 180 may be the portion of the groove 180 having the greatest radius with respect to the longitudinal axis L-L. Flow may enter the groove 180 at the first, upstream end. The groove 180, and any hairstyling formulation/propellant flow therein, spirals inwardly from the first end 190, towards the longitudinal axis L-L. The groove 180 terminates at a second end 191. The second end 191 may be the portion of the groove 180 having the smallest radius with respect to the longitudinal axis L-L.

The flow area of the present invention may be conceptually divided into two flow paths. The first flow path is divided between four discrete grooves 180, and does not circumscribe the longitudinal axis L-L at any particular cross section. The second flow path, contiguous with the first, blends the flow to circumscribe the longitudinal axis L-L at all cross sections from the virtual plane to the nozzle 2. Contrary to the prior art, the projected length of the first flow path, may be less than the projected length of the second flow path, taken parallel to the longitudinal axis L-L.

Referring to Fig. 22, the interface between the four grooves 180 within the housing 136 and the helix cup 130 provides four ports, one corresponding to each groove 180. The ports are the planar projection of the flow area between the second end 191 of the groove 180 and the helix cup 130. Upstream of the ports, the flow is divided into discrete flow paths corresponding to the grooves 180. Downstream of the ports, the four discrete flow paths can intermix and converge in the circumferential direction to form a continuous film and be discharged through the nozzle 2.

The flow in the continuous film of the helix cup 130 circumscribes the longitudinal axis. Further the flow converges in the axial direction, as the nozzle 2 is approached. The flow in the helix cup 130 radially converges in the axial direction. Such radial convergence may be a concave wall 164, a convex wall or a combination thereof.

The converging wall may have some portions 166 which are straight, but the entirety of the wall, from the one or more inlet port(s) to the nozzle 2 is not. By straight, it is meant that a line on the wall from an inlet port 192 to the nozzle 2, forms the hypotenuse of a triangle. As noted above, the triangle has one leg coincident the longitudinal axis and the other leg a radius of the circle connected to the hypotenuse.

In the helix cup 130, flow can intermix and circumscribe the longitudinal axis. As the flow approaches the discharge nozzle 2, the flow may converge. Such convergence increases the density of the flow, creating a low pressure zone. Further, the radius of the flow decreases throughout much of the longitudinal direction, although a portion of constant radius may be included proximate the discharge nozzle 2.

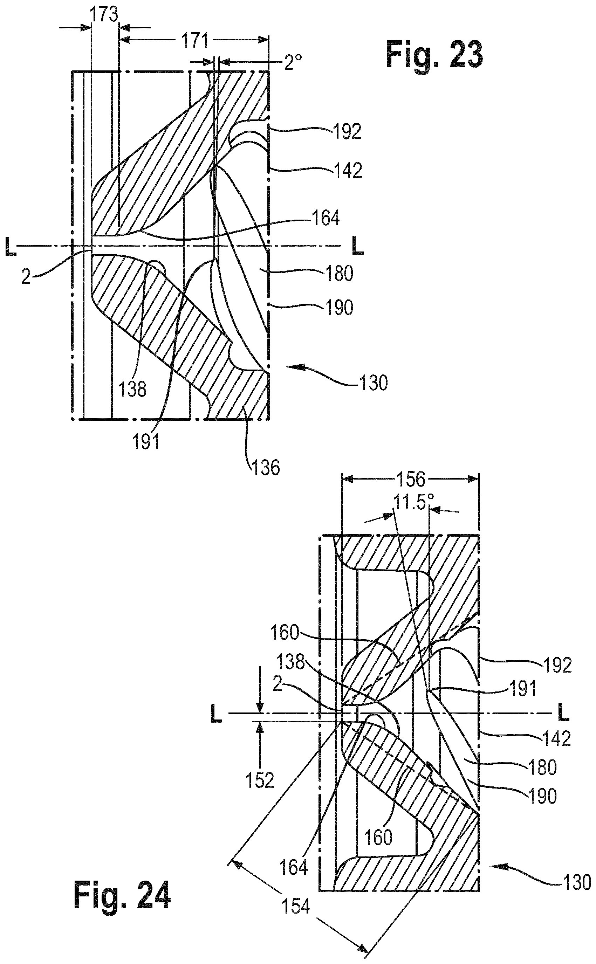

Referring to Figs. 23 and 24, the grooves 180 may be skewed relative to a virtual plane disposed perpendicular to the longitudinal axis. The skew may be constant or may increase as the nozzle 2 is approached. For the embodiments described herein, a skew angle relative to the cutting plane 184 of 2° to 11.5° has been found suitable. If the skew angle changes throughout the length of the groove 180, the skew may increase as the second end 191 of the groove 180 is approached, terminating within the aforementioned skew angle range. The skew angle may be determined between the smallest angle of the vector through the centroid of the groove 180 at the position of the cutting plane 184 and the cutting plane 184. A tighter droplet size distribution has been found to occur with an 11.5° skew angle than with a 2° skew angle.

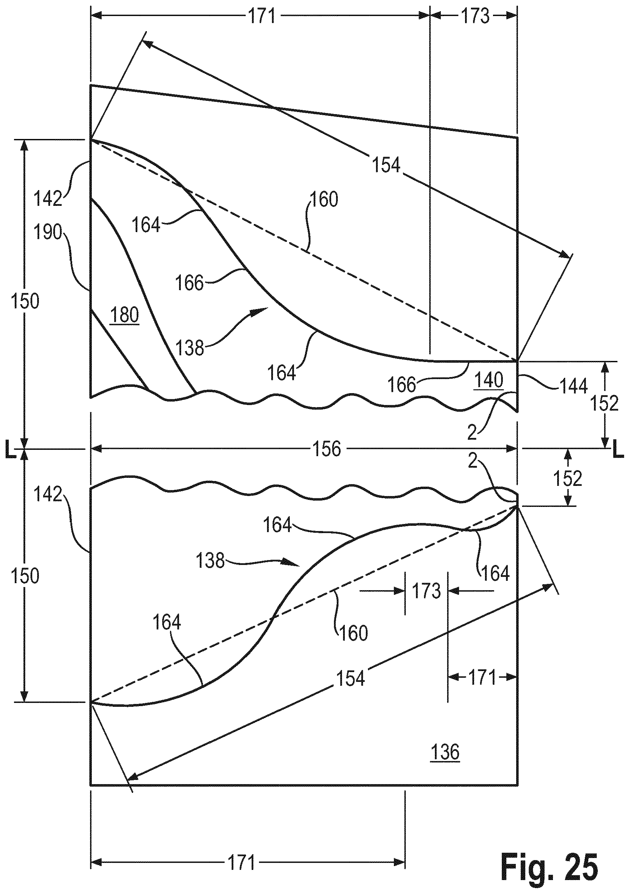

Referring to Fig. 25 in another embodiment, the funnel wall 138 may be partially or completely convexly shaped. In this embodiment, like the previous embodiments, the funnel wall 138 deviates from linearity between the funnel wall 138 inlet 142 and the funnel wall 138 outlet 144 at the nozzle 2. This geometry, like the previous geometries, may have a surface area and subtended volume which do not correspond to the equalities set forth in equations (1) and (2) above.

One of skill will recognize that hybrid geometries are also feasible and within the scope of the claimed invention. In a hybrid embodiment, a portion of the funnel wall 138 may be convex, another portion may be concave, and optionally, yet another portion may be linear. Again, in such a geometry, the funnel wall 138 may have a surface area and subtended volume which do not correspond to the equalities set forth in equations (1) and (2) above.

The embodiments of Fig. 25 show a funnel wall 138 having contiguous concave and convex portions 164 in the convergent portion 171 of that funnel wall 138. The lower embodiment of Fig. 25 further has a concave portion 164 which is not convergent at 173. By concave it is meant that the cross section of the funnel wall 138 taken parallel to the longitudinal axis L-L is outwardly arcuate relative to the hypotenuse 160 joining the edge of the inlet 142 and outlet 144. By convex it is meant that the cross section of the funnel wall 138 taken parallel to the longitudinal axis L-L is inwardly arcuate relative to the hypotenuse 160 joining the edge of the inlet 142 and outlet 144.

More particularly, in the upper portion of Fig. 25, moving longitudinally from the inlet 142 towards the outlet 144, the convergent portion 171 of the funnel wall 138 has a convex portion 164, a straight portion 166 and a concave portion 164. The funnel wall also has a portion 173 of constant cross section and which has straight sidewalls 166.

In the lower portion of Fig. 25, substantially the entire funnel wall 138 is convergent as indicated at portions 171. Moving longitudinally from the inlet 142 towards the outlet 144, the first convergent portion 171 comprises both a convex wall 164 and contiguous concave wall 164. The concave funnel wall 138 inflects to not be convergent as indicated at 173. The funnel wall 138 converges at slightly convex portion 164, to terminate at the nozzle 2 without having a straight portion in the funnel wall 138.

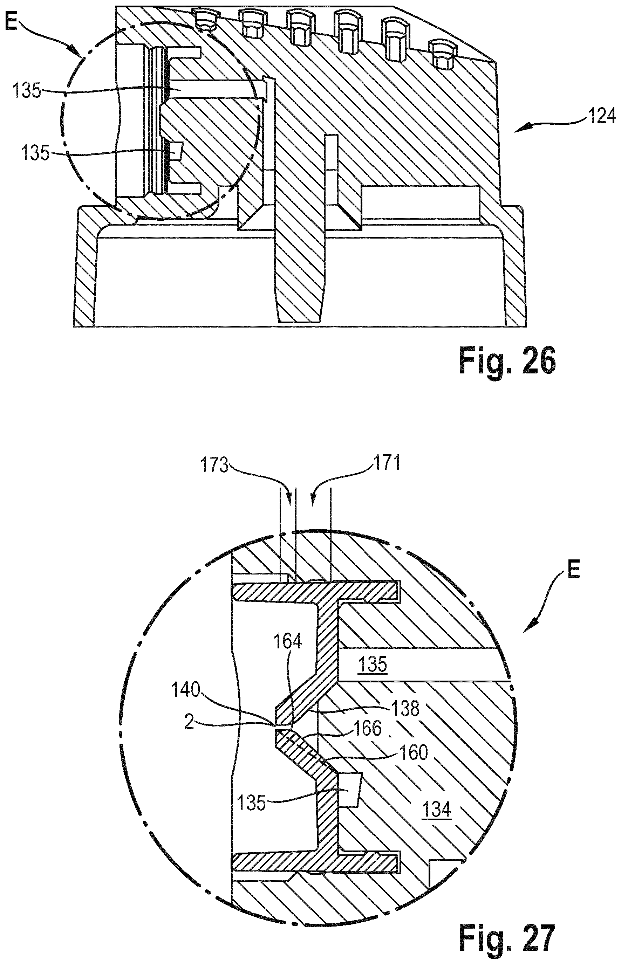

Referring to Figs. 26 and 27, the backstop 134 must be rigid enough to withstand the back pressure encountered during forward spray of the hairstyling formulation from the aerosol hairspray product 120. The backstop 134 must also be able to prevent deflection during assembly of the helix cup 130 to the cap 124. If the backstop 134 deflects during assembly, the helix cup 130 may be inserted too deeply into the cap 124, and proper dispensing may not occur. To prevent this occurrence, a thicker and/or more rigid backstop 134 may be utilized.

Referring particularly to Fig. 27, the backstop 134 may be conically or otherwise convexly shaped. This geometry allows the helix cup 130 to accurately seat during manufacture. Other shapes are suitable as well, so long as a complementary seating surface is presented between the backstop 134 and helix cup 130.

METHOD OF MAKING A HAIRSPRAY

First two solutions are made: a main mix and a second mix. The main mix comprises the hairstyling polymer(s), which are dissolved with stirring in water and components of the preservative system. A second mix is created which comprises water and the paraben-based preservative component(s) (e.g. methyl paraben). Optionally the second mix is heated up in a microwave to 90 to 95°C in order to dissolve the paraben. The two mixes are then combined to create the hairstyling formulation. The hairstyling formulation is then put into the container and then container is sealed by crimping on a sealing mounting cup which includes a valve system. Then the propellant is added under pressure and then the spray nozzle is added to the container.

EXAMPLES

| Examples | 1 | 2 | 3 | 4 | 5 | 6 | 7 | 8 |
|---|---|---|---|---|---|---|---|---|
| Acrylates/hydroxyesters acrylates copolymer [1] | 6.7 | 5.5 | 3.6 | -- | 3.35 | -- | -- | 0.2 |
| Polyurethane-14/AMP-acrylates polymer blend [2] | -- | -- | 3.0 | 10.0 | 6.0 | 10.0 | -- | 6.0 |
| Acrylates Copolymer [3] | -- | -- | -- | 5.6 | -- | 1.7 | 4.1 | 1.0 |
| 2-Aminopropanol (AMP) | 0.6 | 0.25 | 0.17 | 0.35 | 0.15 | 0.2 | 0.3 | 0.25 |
| Castor oil PEG-40 H, (90%) | 0.1 | 0.2 | 0.15 | 0.3 | 0.3 | 0.3 | 0.2 | 0.15 |
| Disodium EDTA | 0.15 | 0.15 | 0.15 | 0.15 | 0.15 | 0.15 | 0.15 | 0.15 |
| Perfume | 0.2 | 0.07 | 0.3 | 0.15 | 0.1 | 0.05 | 0.1 | 0.15 |
| Phenoxyethanol[4] | 0.3 | 0.2 | 0.3 | 0.3 | 0.4 | 0.2 | 0.2 | 0.2 |

(continued)

| Examples | 1 | 2 | 3 | 4 | 5 | 6 | 7 | 8 |
|---|---|---|---|---|---|---|---|---|
| 1,3-bis(hydroxymethyl)-5,5-dimethylimidazolidine-2,4-dione [5] | -- | 0.1 | 0.2 | 0.4 | 0.2 | 0.3 | 0.4 | 0.3 |
| Methylparaben [6] | 0.2 | -- | -- | -- | 0.2 | 0.2 | 0.2 | 0.2 |
| Deionised water | Add to 100 | Add to 100 | Add to 100 | Add to 100 | Add to 100 | Add to 100 | Add to 100 | Add to 100 |

| Examples | 9 | 10 | 11 | 12 | 13 | 14 | 15 | 16 |
|---|---|---|---|---|---|---|---|---|
| Acrylates/hydroxyesters acrylates copolymer [1] | 4.69 | 4.8 | 4.0 | -- | -- | 2.0 | 3.6 | -- |
| Polyurethane-14/AMP-acrylates polymer blend [2] | -- | -- | 7.0 | -- | 7.0 | 6.0 | 7.0 | -- |
| Acrylates Copolymer [3] | -- | -- | -- | 5.1 | 3.9 | -- | -- | 6.5 |
| 2-Aminopropanol (AMP) | 0.42 | 0.49 | 0.4 | 0.38 | 0.25 | 0.21 | 0.37 | 0.53 |
| Castor oil PEG-40 H, (90%) | 0.1 | 0.2 | 0.3 | 0.3 | 0.1 | 0.3 | 0.2 | 0.3 |
| Disodium EDTA | 0.07 | -- | -- | -- | 0.07 | 0.1 | 0.1 | 0.1 |
| Perfume | 0.07 | 0.1 | 0.07 | 0.15 | 0.05 | 0.035 | 0.03 | 0.08 |
| Phenoxyethanol [4] | 0.3 | 0.3 | 0.3 | 0.3 | 0.3 | 0.3 | 0.3 | 0.3 |
| 1,3-bis(hydroxymethyl)-5,5-dimethylimidazolidine-2,4-dione [5] | -- | 0.29 | 0.29 | 0.29 | -- | 0.29 | 0.29 | 0.29 |
| Methylparaben [6] | 0.1 | -- | -- | -- | 0.1 | 0.14 | 0.14 | 0.2 |
| Ethanol | -- | -- | -- | 1 | -- | -- | -- | -- |
| DME | 30 | 30 | 30 | 28 | 30 | 30 | 30 | 40 |
| Deionised water | Add to 100 | Add to 100 | Add to 100 | Add to 100 | Add to 100 | Add to 100 | Add to 100 | Add to 100 |

Key: [1] = Acudyne® 1000 (45% solution); [2] = DynamX $H_2O$® (25% solution); [3] = Balance® CR (45% solution); [4] = Euxyl® PE 9010; [5] = Nipaguard® DMDMH; [6] = PHB-methylester from Schütz. Any of examples 1 to 8 may be placed in a predominantly plastic or predominantly metal container. The propellant may be a compressed gas propellant such that the product comprises 15% or less VOC by total weight of the hairstyling formulation and propellant. The spraying device preferably comprises (I) and/or (II) as described herein.

Examples 9 to 16 are included for comparative purposes.

Any of examples 9 to 16 may be placed in container wherein the container wall comprises at least 80% metal material by total weight of the container. The metal material may be selected from the group consisting of: aluminium, tin plated steel, and combinations thereof. The propellant is DME as stated in the table.

PERFORMANCE DATA

Experiment 1 - Sensory Data

Aerosol hairspray products pursuant to the present invention and selected from the above example section were compared with a standard aerosol hairstyling product with excellent performance. The standard aerosol hairspray product comprises: 50% DME propellant, the container is a metal container, a hairspray formulation comprising 3% Amphomer as hairstyling polymer, circa 38% ethanol, and less than 1% water; and wherein the product comprises VOC 95% (these percentages are by total weight of the hairstyling formulation and propellant).

The ejected compositions from these products are sprayed onto hair and compared for sensory criteria. When the difference between the product pursuant to the present invention and the standard product is from -1 to +1 (i.e. one point better or worse), then an equals sign (=) is marked. When the difference is less than -1 or greater than +1 then a - or a + is marked, respectively. When the difference is greater than +2, then a ++ is marked.

| Criteria | Ex. 4 | Ex. 4 | Ex. 5 | Ex. 9 | Ex. 13 |
|---|---|---|---|---|---|
| Spraying device | Comprises (I) as described herein. | Comprises (II) as described herein. | Comprises (II) as described herein. | Comprises a vapour housing hole. | Comprises a vapour housing hole. |
| Approx.VOC (%) | <1 | <1 | <1 | 30 | 30 |
| Propellant | Nitrogen | Nitrogen | Nitrogen | DME | DME |
| Total hairstyling polymer amount (%) | 5 | 5 | 3 | 3 | 5 |
| INITIAL HOLD [1] | = | + | = | = | = |
| DRYING TIME [2] | = | = | = | = | = |
| FEEL [3] | ++ | ++ | ++ | ++ | ++ |
| LOOK [4] | = | = | = | = | = |
| LOOK [5] | = | = | = | = | = |
| FEEL [6] | = | - | = | = | = |
| FEEL [7] | = | = | = | = | = |
| Key: [1] = Hold on mannequin (least hold to most hold); = Humidity after application (hair feels very dry to hair feels very wet); [3] = Stickiness of hands/hair (not sticky at all to very sticky); [4] = Hair look mannequin (very natural to very clumped); [5] = : Residues on mannequin hair (no residues at all to lots of residues); [6] = Hair feel of mannequin after combing (very rough to very smooth); [7] = Drawing fingers through hair of mannequin (hair clumped together to hair free flowing). | | | | | |

Experiment 2 - Technical Data

The setting and the hold conferred to a hairstyle by a composition can be determined by measuring, respectively, the 3-point bending force and the hold force factor. 3-point bending force methodology: 0.5 ml/g hairstyling formulation is applied to the hair tress and massaged in for 1 min. The hair tresses are then dried in a drying cabinet for 45 min at 45°C. The tresses are then smoothed over by the fingers and dried overnight in a chamber at 20°C at 65% relative humidity. The measurement is made with stamp at 5 positions on the sample. The 3-point bending force is measured according to the methodology detailed in F. Frosch, F. Vogel, 6th International Hair Science Symposium Of the German Wool Research Institute, Luneburg/Germany (1988). See also the methodology DIN-EN-658-5 from the American National Standards Institute. A mean value is calculated after 9 repeats are performed (i.e. n = 9).

Hold force factor (also known as curl retention) methodology: 0.5 ml/g hairstyling formulation is applied to the hair tress and massaged in for 1 min. Each hair tress is then adjusted to 50% by weight of the hair tress and combed three times. The tresses are plaited and dried in a drying cabinet for 45 min at 45°C. The tresses are then dried overnight in a climatic chamber at 20°C at 65% relative humidity. The curl retention measurements are taken the following day. The climatic conditions are: 20°C at 85% relative humidity. The reading times are: after 0 h, 1 h, 2h, 3 h 5 h and 24 h (h means hour). The hold force factor is measured according to the methodology detailed in C.R. Robbins, Chemical and Physical Behavior of Human Hair, 3rd edition, page 352, Springer-Verlag, New York (1994). A mean value is calculated after 3 repeats are performed (i.e. n = 3).

Table X details the 3-point bending force and the hold factor after 1h, 5h and 24h of the hairstyle, after applying the below-detailed hairstyling formulations. Where indicated, the hairstyling formulation is as per an example from the table in the examples section above. Samples A to D were treated with hairstyling formulations A to D, which comprise the indicated hairstyling polymer in deionised water. Total hairstyling polymer present is indicated in brackets. Samples α to γ were control treatments as detailed below.

Table X

| Parameters \ Sample | Comp. Ex. A | B | Comp. Ex. C | D | Ex. 1 | Ex. 4 | α | β | γ |
|---|---|---|---|---|---|---|---|---|---|
| Hairstyling polymer [wt% of total hairstyling polymer] | 5 [3%] | 2 [3% ] | 3 [3%] | Mixture of [1] and [2] (1:1 ratio) [3%] | 1 [3%] | Mixture of [3] and [2] (1.1 ratio) [5%] | N/T | 5 [3%] | 6 [3%] |
| 3-point bending force (N) 1 break - hold | 2.101 +/- 0.413 | 2.48 +/- 0.60 | 3.02 +/- 0.70 | 2.216 +/- 0.449 | 2.231 +/- 0.358 | 3.211 +/- 0.796 | 0.09 +/- 0.01 | 1.82 +/- 0.62 | 1.95 +/- 0.628 |
| 3-point bending force (%) 3rd break - elasticity | 50.70 +/-7.46 | 55.53 +/-8.13 | 41.55 +/-3.53 | 43.33 +/- 4.13 | 57.61 +/- 5.32 | 41.34 +/- 6.87 | 92.76 +/- 19.32 | 49.97 +/- 11.00 | 51.81 +/- 12.56 |
| Hold factor (%) after 0 h | 88.17 +/-0.13 | 91.814 +/-1.791 | 90.72+/- 5.59 | 94.00 +/- 1.79 | 95.02 +/- 1.46 | 91.34 +/- 4.58 | 77.37 +/- 1.94 | 89.66 +/- 4.12 | 90.65 +/- 1.83 |
| Hold factor (%) after 1 h | 70.95 +/-4.64 | 82.73 +/-2.026 | 74.6 +/- 4.88 | 83.39 +/- 0.90 | 85.25 +/- 2.44 | 82.95 +/- 6.92 | 30.98 +/- 1.87 | 55.46 +/- 2.59 | 52.21 +/- 8.99 |
| Hold factor (%) after 5 h | 54.18 +/-7.83 | 70.77 +/-6.45 | 57.61 +/-4.89 | 71.94 +/- 1.82 | 76.35 +/- 2.75 | 67.89 +/- 3.92 | 10.12 +/- 0.51 | 16.58 +/- 1.97 | 14.72 +/- 1.52 |
| Hold factor (%) after 24 h | 50.75 +/-7.71 | 60.9 +/- 3.899 | 51.04 +/- 4.7 | 67.31 +/- 1.16 | 73.58 +/- 2.64 | 65.19 +/- 7.10 | 7.50 +/- 1.08 | 12.53 +/- 2.94 | 11.51 +/- 1.08 |
| Key: [1] = Acudyne® 1000; [2] = DynamX® H2O; [3] = Balance® CR; [4] = Amphomer®; N/T = not treated; [5] = PVP/VA (vinylpyrrolidone/vinylacetate copolymer) 64; [6] = PVP (polyvinylpyrrolidone) K30. | | | | | | | | | |

Conclusions from experiment 2 include: the samples left untreated exhibited the weakest (i.e. lowest) 3-point bending force and hold factor. PVP VA 64 and PVP K30 are softer hairstyling polymers, which, in the context of the hairstyling formulation pursuant to the present invention provide weaker hold as well as lower humidity resistance. The hard hairstyling polymers, especially the blends, show high humidity resistance - see hold factor values after 24 h in table X.

**Claims**

1.  An aerosol hairspray product (120) for styling and/or shaping hair wherein the product (120) comprises:

    i. a container comprising a container wall which encloses a reservoir (122) for storing a hairstyling formulation and a propellant;
    ii. the hairstyling formulation comprising:

    (a) at least 50% water by total weight of the hairstyling formulation and propellant; and
    (b) from 0.01% to 20% of a hairstyling polymer by total weight of the hairstyling formulation and propellant, wherein the hairstyling polymer is selected from the group consisting of: acrylates copolymers of two or more monomers of (meth)acrylic acid or one of their simple esters; octylacrylamide/acrylate/butylaminoethyl methacrylate copolymers; acrylates/hydroxyesters acrylates copolymers of butyl acrylate, methyl methacrylate, methacrylic acid, ethyl acrylate and hydroxyethyl methacrylate; polyurethane-14/AMP-acrylates polymer blend; and mixtures thereof; wherein the hairstyling polymer is at least 60%, or at least 80% neutralized; and

iii. a propellant, which is selected from the group consisting of compressed gas propellants, liquefied gas propellants, and mixtures thereof; and
iv. a spraying device attached to the container for dispensing the hairstyling formulation from the reservoir (122) of the container;

and wherein the product (120) comprises 2% or less alcohol by total weight of the hairstyling formulation and propellant, or is less than 1% of alcohol;
wherein the product (120) comprises less than 15% volatile organic compound by total weight of the hairstyling formulation and propellant.

2. The product (120) according to claim 1, wherein the hairstyling formulation comprises a surfactant, wherein the surfactant is selected from the group consisting of cationic surfactants, non-ionic surfactants, anionic surfactants, and mixtures thereof.

3. The product (120) according to any preceding claim, wherein the kinematic viscosity, measured according to standard test DIN EN ISO 3104, of the hairstyling formulation is from 1 mm$^2$/s to 25 mm$^2$/s, or from 1 mm$^2$/s to 15 mm$^2$/s, or from 2 mm$^2$/s to 10 mm$^2$/s, or from 1 mm$^2$/s to 4 mm$^2$/s, or from 1.2 mm$^2$/s to 3 mm$^2$/s.

4. The product (120) according to any preceding claim, comprising from 0.01% to 16%, or from 0.01% to 10%, or from 1% to 8%, or from 2% to 6% of the hairstyling polymer, by total weight of the hairstyling formulation and propellant.

5. The product (120) according to any preceding claim, wherein the product (120) comprises less than 1% of volatile organic compound, by total weight of the hairstyling formulation and propellant.

6. The product (120) according to claim 5, wherein the propellant is a compressed gas propellant, wherein the compressed gas propellant is selected from the group consisting of air, nitrogen, nitrous oxide, carbon dioxide, and mixtures thereof.

7. The product (120) according to any of claims 5 to 6, wherein the pressure inside the container is from 6 bar to 12 bar, or from 8 bar to 10 bar, at 50°C.

8. The product (120) according to any preceding claim, wherein the spraying device comprises:

(I) a spray nozzle (2) for atomizing the hairstyling formulation, said nozzle (2) comprising
a fluid chamber (40), preferably a ring chamber, for receiving the hairstyling formulation from the reservoir (122) of the container, at least one feeding channel (42) for feeding the hairstyling formulation from the fluid chamber (40) radially inward into a swirl chamber (44) and an outlet channel (18) with an entrance end (54) facing the swirl chamber (44) and an exit end (56) for atomizing the hairstyling formulation to the environment of the spray nozzle (2), wherein the outlet channel (18) tapers in the dispensing direction of the hairstyling formulation, and/or (II)

(a) a spray nozzle (2) for atomizing the hairstyling formulation, said nozzle (2) defining an axial direction and having a longitudinal axis (L-L) therethrough;
(b) at least one discrete inlet port, said inlet port having an associated inlet area, said inlet port not circumscribing said longitudinal axis (L-L) and being radially offset therefrom;
(c) a flow area joining said inlet port and said nozzle (2), said flow area comprising a surface of revolution said longitudinal axis (L-L), said surface of revolution convergently directing flow from said at least one inlet port to said nozzle (2);

wherein at least a portion of the surface of revolution circumscribing said longitudinal axis (L-L) is curvilinear.

9. The product (120) according to claim 8, wherein according to (I) the outlet channel (18) tapers steadily or/and a tapering portion of the outlet channel (18) abuts the exit end (56), the tapering portion preferably abutting the entrance end (54) as well.

10. The product (120) according to claim 8, wherein the surface of revolution according to (II) has at least a portion of which is concave and/or convex relative to the longitudinal axis (L-L).

**11.** The product (120) according to any preceding claim, wherein the container wall comprises at least 80% plastic material by total weight of the container.

**12.** The product (120) according to claim 11, wherein the plastic material is selected from the group consisting of polyolefins, polyesters, polyamide, polyvinylchloride, acrylic, polycarbonates, polyethylene naphthalate, polyethylene therephthalate, polystyrene, polyurethane, and mixtures thereof; or polyethylene terephthalate, polyethylene napththalate, and mixtures thereof.

**13.** The product (120) according to any of claims 1 to 4, wherein the container wall comprises at least 80% metal material by total weight of the container, and wherein the metal material is selected from the group consisting of: aluminium, tin plated steel, and combinations thereof; and wherein the propellant is a liquefied gas propellant, and wherein the liquefied gas propellant is selected from the group consisting of dimethylether, 1,1-difluoroethane, 1,1,1,2-tetrafluoroethane, pentane, *n*-butane, *iso*-butane, propane, *trans*-1,3,3,3-tetrafluoropropene, and mixtures thereof; or dimethylether, 1,1-difluoroethane, and mixtures thereof.

**14.** A method for styling hair comprising:

i. applying to hair an ejected composition, wherein the ejected composition is ejected by the hairspray product (120) according to any of the preceding claims;
ii. drying the ejected composition on the hair.

**15.** The use of the product (120) according to any of claims 1 to 13, for fixing and/or shaping a hairstyle.

**Patentansprüche**

**1.** Aerosol-Haarsprayprodukt (120) zum Frisieren und/oder Formen von Haar, wobei das Produkt (120) umfasst:

i. einen Behälter, umfassend eine Behälterwand, die ein Reservoir (122) zum Aufbewahren einer Haarfrisierformulierung und eines Treibstoffs einschließt,
ii. wobei die Haarfrisierformulierung umfasst:

(a) zu mindestens 50 Gew.-% Wasser, bezogen auf das Gesamtgewicht der Haarfrisierformulierung und des Treibmittels; und
(b) zu von 0,01 Gew.-% bis 20 Gew.-% ein Haarfrisierpolymer, bezogen auf das Gesamtgewicht der Haarfrisierformulierung und des Treibmittels, wobei das Haarfrisierpolymer ausgewählt ist aus der Gruppe, bestehend aus: Acrylat-Copolymeren von zwei oder mehr Monomeren von (Meth)acrylsäure oder einem ihrer einfachen Ester; Octylacrylamid/Acrylat/Butylaminoethylmethacrylat-Copolymeren; Acrylaten/Hydroxyestern Acrylaten Copolymeren von Butylacrylat, Methylmethacrylat, Methacrylsäure, Ethylacrylat und Hydroxyethylmethacrylat; Polyurethan-14/AMP-Acrylaten-Polymermischung; und Mischungen davon; wobei das Haarfrisierpolymer mindestens zu 60 % oder mindestens zu 80 % neutralisiert ist; und

iii. ein Treibmittel, das ausgewählt ist aus der Gruppe, bestehend aus Druckgastreibmitteln, Flüssiggastreibmitteln und Mischungen davon; und
iv. eine Sprühvorrichtung, die an dem Behälter zum Abgeben der Haarfrisierformulierung aus dem Reservoir (122) des Behälters befestigt ist;

und wobei das Produkt (120) zu 2 Gew.-% oder weniger Alkohol, bezogen auf das Gesamtgewicht der Haarfrisierformulierung und des Treibmittels, umfasst, oder weniger als 1 % Alkohol beträgt;
wobei das Produkt (120) zu weniger als 15 Gew.-% flüchtige organische Verbindung, bezogen auf das Gesamtgewicht der Haarfrisierformulierung und des Treibmittels, umfasst.

**2.** Produkt (120) nach Anspruch 1, wobei die Haarfrisierformulierung ein Tensid umfasst, wobei das Tensid ausgewählt ist aus der Gruppe bestehend aus kationischen Tensiden, nichtionischen Tensiden, anionischen Tensiden und Mischungen davon.

**3.** Produkt (120) nach einem der vorstehenden Ansprüche, wobei die kinematische Viskosität der Haarfrisierformulierung, gemessen nach der Normprüfung DIN EN ISO 3104, von 1 mm$^2$/s bis 25 mm$^2$/s oder von 1 mm$^2$/s bis 15

mm$^2$/s oder von 2 mm$^2$/s bis 10 mm$^2$/s oder von 1 mm$^2$/s bis 4 mm$^2$/s oder von 1,2 mm$^2$/s bis 3 mm$^2$/s beträgt.

4. Produkt (120) nach einem der vorstehenden Ansprüche, umfassend, bezogen auf das Gesamtgewicht der Haarfrisierformulierung und des Treibstoffs, zu von 0,01 % bis 16 % oder zu von 0,01 % bis 10 % oder zu von 1 % bis 8 % oder zu von 2 % bis 6 % das Haarfrisierpolymer.

5. Produkt (120) nach einem der vorstehenden Ansprüche, wobei das Produkt (120), zu weniger als 1 Gew.-% flüchtige organische Verbindung, bezogen auf das Gesamtgewicht der Haarfrisierformulierung und des Treibstoffs, umfasst.

6. Produkt (120) nach Anspruch 5, wobei das Treibmittel ein Druckgastreibmittel ist, wobei das Druckgastreibmittel ausgewählt ist aus der Gruppe bestehend aus Luft, Stickstoff, Stickstoffoxid, Kohlendioxid und Mischungen davon.

7. Produkt (120) nach einem der Ansprüche 5 bis 6, wobei der Druck innerhalb des Behälters von 6 bar bis 12 bar oder von 8 bar bis 10 bar bei 50 °C beträgt.

8. Produkt (120) nach einem der vorstehenden Ansprüche, wobei die Sprühvorrichtung umfasst:

(I) eine Sprühdüse (2) zum Zerstäuben der Haarfrisierformulierung, die Düse (2) umfassend eine Fluidkammer (40), vorzugsweise eine Ringkammer, zum Aufnehmen der Haarfrisierformulierung aus dem Reservoir (122) des Behälters, mindestens einen Zuführkanal (42) zum Zuführen der Haarfrisierformulierung aus der Fluidkammer (40), radial nach innen in eine Wirbelkammer (44), und einen Auslasskanal (18) mit einem Eintrittsende (54), das der Wirbelkammer (44) zugewandt ist, und einem Austrittsende (56) zum Zerstäuben der Haarfrisierformulierung in die Umgebung der Sprühdüse (2), wobei sich der Auslasskanal (18) in der Abgaberichtung der Haarfrisierformulierung verjüngt, und/oder
(II)

(a) eine Sprühdüse (2) zum Zerstäuben der Haarfrisierformulierung, wobei die Düse (2) eine axiale Richtung definiert und eine Längsachse (L-L) aufweist, die durch sie hindurch verläuft;
(b) mindestens eine separate Einlassöffnung, wobei die Einlassöffnung einen dazugehörigen Einlassbereich aufweist, wobei die Einlassöffnung nicht die Längsachse (L-L) umschreibt und radial davon versetzt ist;
(c) einen Fließbereich, der an die Einlassöffnung und die Düse (2) anschließt, wobei der Fließbereich eine Umdrehungsoberfläche um die Längsachse (L-L) herum umfasst, wobei die Umdrehungsoberfläche den Fluss konvergierend von der mindestens einen Einlassöffnung zu der Düse (2) leitet; wobei wenigstens ein Teil der Umdrehungsoberfläche, welche die Längsachse (L-L) umschreibt, krummlinig ist;

9. Produkt (120) nach Anspruch 8, wobei gemäß (I) der Auslasskanal (18) sich stetig verjüngt oder/und ein sich verjüngender Abschnitt des Auslasskanals (18) an das Austrittsende (56) angrenzt, wobei der sich verjüngende Abschnitt vorzugsweise auch an das Eintrittsende (54) angrenzt.

10. Produkt (120) nach Anspruch 8, wobei die Umdrehungsoberfläche gemäß (II) mindestens einen Abschnitt aufweist, der in Bezug zu der Längsachse (L-L) konkav und/oder konvex ist.

11. Produkt (120) nach einem der vorstehenden Ansprüche, wobei die Behälterwand, bezogen auf das Gesamtgewicht des Behälters, zu mindestens 80 % Kunststoffmaterial umfasst.

12. Produkt (120) nach Anspruch 11, wobei das Kunststoffmaterial ausgewählt ist aus der Gruppe, bestehend aus Polyolefinen, Polyestern, Polyamid, Polyvinylchlorid, Acryl, Polycarbonaten, Polyethylennaphthalat, Polyethylenterephthalat, Polystyrol, Polyurethan und Mischungen davon; oder Polyethylenterephthalat, Polyethylennapththalat und deren Mischungen.

13. Produkt (120) nach einem der Ansprüche 1 bis 4, wobei die Behälterwand zu mindestens 80 Gew.-%, bezogen auf das Gesamtgewicht des Behälters, Metallmaterial umfasst, und wobei das Metallmaterial ausgewählt ist aus der Gruppe, bestehend aus: Aluminium, verzinntem Stahl und Kombinationen davon; und wobei das Treibmittel ein verflüssigtes Treibgas ist, und wobei das verflüssigte Treibgas ausgewählt ist aus der Gruppe, bestehend aus Dimethylether, 1,1-Difluorethan, 1,1,1,2-Tetrafluorethan, Pentan, *n*-Butan, *iso*-Butan, Propan, *trans*-1,3,3,3-Tetrafluorpropen und Mischungen davon; oder Dimethylether, 1,1-Difluorethan und Mischungen davon.

14. Verfahren zum Frisieren von Haar, umfassend:

i. Auftragen einer ausgeworfenen Zusammensetzung auf Haar, wobei die ausgeworfene Zusammensetzung durch das Haarsprayprodukt (120) nach einem der vorstehenden Ansprüche ausgeworfen wird;
ii. Trocknen der ausgeworfenen Zusammensetzung auf dem Haar.

15. Verwendung des Produkts (120) nach einem der Ansprüche 1 bis 13 zum Fixieren und/oder Formen einer Frisur.


**Revendications**

1. Produit fixatif capillaire en aérosol (120) pour le coiffage et/ou la mise en forme des cheveux, où le produit (120) comprend :

   i. un récipient comprenant une paroi de récipient qui entoure un réservoir (122) pour stocker une formulation de coiffage et un propulseur ;
   ii. la formulation de coiffage comprenant :

   (a) au moins 50 % d'eau en poids total de la formulation de coiffage et du propulseur ; et
   (b) de 0,01 % à 20 % d'un polymère de coiffage en poids total de la formulation de coiffage et du propulseur, dans lequel le polymère de coiffage est choisi dans le groupe constitué de : copolymère d'acrylates de deux ou plus monomères d'acide (méth)acrylique ou d'un de leurs esters simples ; copolymères d'octylacrylamide/acrylate/méthacrylate de butylaminoéthyle ; copolymères acrylates/acrylates d'hydroxyesters d'acrylate de butyle, de méthacrylate de méthyle, d'acide méthacrylique, d'acrylate d'éthyle et de méthacrylate d'hydroxyéthyle ; mélange polymère polyuréthane-14/AMP-acrylates ; et leurs mélanges ; dans laquelle le polymère de coiffage est neutralisé à au moins 60 %, ou au moins 80 % ; et

   iii. un propulseur, qui est choisi dans le groupe constitué de gaz propulseurs comprimés, gaz propulseurs liquéfiés, et leurs mélanges ; et
   iv. un dispositif de pulvérisation fixé au récipient pour distribuer la formulation de coiffage à partir du réservoir (122) du récipient ;

   et dans lequel le produit (120) comprend 2 % ou moins d'alcool en poids total de la formulation de coiffage et du propulseur, ou moins de 1 % de d'alcool ;
   dans lequel le produit (120) comprend moins de 15 % de composé organique volatil, en poids total de la formulation de coiffage et du propulseur.

2. Produit (120) selon la revendication 1, dans lequel la formulation de coiffage comprend un agent tensioactif, dans lequel l'agent tensioactif est choisi dans le groupe constitué d'agents tensioactifs cationiques, agents tensioactifs non ioniques, agents tensioactifs anioniques, et des mélanges de ceux-ci.

3. Produit (120) selon l'une quelconque des revendications précédentes, dans lequel la viscosité cinématique, mesurée selon le test normalisé DIN EN ISO 3104, de la formulation de coiffage va de 1 mm$^2$/s à 25 mm$^2$/s, ou de 1 mm$^2$/s à 15 mm$^2$/s, ou de 2 mm$^2$/s à 10 mm$^2$/s, ou de 1 mm$^2$/s à 4 mm$^2$/s, ou de 1,2 mm$^2$/s à 3 mm$^2$/s.

4. Produit (120) selon l'une quelconque des revendications précédentes, comprenant de 0,01 % à 16 %, ou de 0,01 % à 10 %, ou de 1 % à 8 %, ou de 2 % à 6 % du polymère de coiffage, en poids total de la formulation de coiffage et du propulseur.

5. Produit (120) selon une quelconque revendication précédente, dans lequel le produit (120) comprend moins de 1 % de composé organique volatil, en poids total de la formulation de coiffage et du propulseur.

6. Produit (120) selon la revendication 5, dans lequel le propulseur est un gaz propulseur comprimé, dans lequel le gaz propulseur comprimé est choisi dans le groupe constitué d'air, azote, oxyde nitreux, dioxyde de carbone et leurs mélanges.

7. Produit (120) selon l'une quelconque des revendications 5 à 6, dans lequel la pression à l'intérieur du récipient va de 6 bar à 12 bar, ou de 8 bar à 10 bar, à 50 °C.

8. Produit (120) selon l'une quelconque des revendications précédentes, dans lequel le dispositif de pulvérisation comprend :

(I) une buse de pulvérisation (2) pour atomiser la formulation de coiffage, ladite buse (2) comprenant une chambre à fluide (40), de préférence une chambre annulaire, pour recevoir la formulation de coiffage à partir du réservoir (122) du récipient, au moins un canal d'alimentation (42) pour alimenter la formulation de coiffage à partir de la chambre à fluide (40) radialement vers l'intérieur dans une chambre de tourbillonnement (44) et un canal de sortie (18) avec une extrémité d'entrée (54) faisant face à la chambre de tourbillonnement (44) et une extrémité de sortie (56) pour atomiser la formulation de coiffage vers l'environnement de la buse de pulvérisation (2), dans lequel le canal de sortie (18) s'effile dans la direction de distribution de la formulation de coiffage, et/ou

(II)

(a) une buse de pulvérisation (2) pour atomiser la formulation de coiffage, ladite buse (2) définissant une direction axiale et possédant un axe longitudinal (L-L) à travers celle-ci ;
(b) au moins un orifice d'entrée distinct, ledit orifice d'entrée possédant une zone d'entrée associée, ledit orifice d'entrée n'entourant pas ledit axe longitudinal (L-L) et étant décalé en sens radial de celle-ci ;
(c) une zone d'écoulement reliant ledit orifice d'entrée et ladite buse (2), ladite zone d'écoulement comprenant une surface de révolution autour dudit axe longitudinal (L-L), ladite surface de révolution dirigeant de façon convergente l'écoulement dudit au moins un orifice d'entrée vers ladite buse (2) ; dans lequel au moins une partie de la surface de révolution entourant ledit axe longitudinal (L-L) est curviligne.

9. Produit (120) selon la revendication 8, dans lequel, selon (I), le canal de sortie (18) s'effile régulièrement ou/et une partie qui s'effile du canal de sortie (18) vient en butée contre l'extrémité de sortie (56), la partie qui s'effile venant de préférence en butée contre l'extrémité d'entrée (54) également.

10. Produit (120) selon la revendication 8, dans lequel la surface de révolution selon (II) a au moins une partie de celle-ci qui est concave et/ou convexe par rapport à l'axe longitudinal (L-L).

11. Produit (120) selon l'une quelconque des revendications précédentes, dans lequel la paroi de récipient comprend au moins 80 % de matériau en plastique en poids total du récipient.

12. Produit (120) selon la revendication 11, dans lequel le matériau en plastique est choisi dans le groupe constitué de polyoléfines, polyesters, polyamide, poly(chlorure de vinyle), acrylique, polycarbonates, naphtalate de polyéthylène, téréphtalate de polyéthylène, polystyrène, polyuréthane, et leurs mélanges ; ou téréphtalate de polyéthylène, naphtalate de polyéthylène et des mélanges de ceux-ci.

13. Produit (120) selon l'une quelconque des revendications 1 à 4, dans lequel la paroi de récipient comprend au moins 80 % de matériau métallique en poids total du récipient, et dans lequel le matériau métallique est choisi dans le groupe constitué de : aluminium, acier étamé, et des combinaisons de ceux-ci ; et dans lequel le propulseur est un gaz propulseur liquéfié, et dans lequel le gaz propulseur liquéfié est choisi dans le groupe constitué d'éther diméthylique, 1,1-difluoréthane, 1,1,1,2-tétrafluoroéthane, pentane, n-butane, iso-butane, propane, *trans*-1,3,3,3-tétrafluoropropène, et leurs mélanges ; ou éther diméthylique, 1,1-difluoréthane, et des mélanges de ceux-ci.

14. Procédé de coiffage des cheveux, comprenant :

i. l'application sur les cheveux d'une composition éjectée, dans lequel la composition éjectée est éjectée par le produit fixatif capillaire (120) selon l'une quelconque des revendications précédentes ;
ii. le séchage de la composition éjectée sur les cheveux.

15. Utilisation du produit (120) selon l'une quelconque des revendications 1 à 13, pour la fixation et/ou la mise en forme d'une coiffure.

Fig. 1

Fig. 2

## Fig. 3

## Fig. 4

# Fig. 5

# Fig. 6

Fig. 7

Fig. 8

Fig. 9

Fig. 10

Fig. 11

## Fig. 12

124

138

136

130
140

135  134

D

## Fig. 13

136

D

130

144

140

138

135

134

## Fig. 14

5A  136

130

142

144

138

140

5A

Fig. 15

Fig. 16

Fig. 17

## Fig. 18

## Fig. 19

**Fig. 20**

**Fig. 21**

**Fig. 22**

Fig. 23

Fig. 24

Fig. 25

**Fig. 26**

**Fig. 27**

**EP 3 260 110 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090104138 A1
- EP 0758545 A1
- DE 29707765 U1
- US 5304368 A1
- US 3819090 A
- US 5199615 A

### Non-patent literature cited in the description

- **F. FROSCH ; F. VOGEL.** *6th International Hair Science Symposium Of the German Wool Research Institute,* 1988
- **C.R. ROBBINS.** Chemical and Physical Behavior of Human Hair. Springer-Verlag, 1994, 352